# EUROPÄISCHE PATENTANMELDUNG

(11) **EP 4 814 152 A1**
(43) Veröffentlichungstag der Anmeldung: **30.09.2026**
(21) Anmeldenummer: 25167046.9
(22) Anmeldetag: 28.03.2025
(51) Int. Cl.: C22C 33/04, C21C 5/28, C21C 5/52, G16C 20/30, G16C 60/00, C21C 5/46

(54) **VERFAHREN UND SYSTEM ZUR HERSTELLUNG VON VERGÜTUNGSSTÄHLEN FÜR ÖLFELDANWENDUNGEN**

(71) Anmelder: voestalpine Tubulars GmbH & Co. KG, 8652 Kindberg-Aumühl (AT); voestalpine Stahl Donawitz GmbH, 8700 Leoben-Donawitz (AT)
(72) Erfinder: Klarner, Jürgen, 8652 Kindberg-Aumühl (AT); Sonnleitner, Robert, 8652 Kindberg-Aumühl (AT); Klösch, Gerald, 8700 Leoben (AT); Karner, Peter, 8700 Leoben (AT)
(74) Vertreter: WSL Patentanwälte Partnerschaft mbB

(57) **Zusammenfassung**

Die Erfindung betrifft ein Verfahren zur Herstellung von Vergütungsstählen für Ölfeldanwendungen mit spezifizierten werkstoffkundlichen Eigenschaften, welche durch eine Ziellegierung sichergestellt werden, wobei zumindest ein primärmetallurgisches Aggregat zu einer ersten Schmelze führt, wobei aus der ersten Schmelze zumindest eine Analyse von Gehalten der Elemente durchgeführt wird, wobei zumindest ein Ist-Wert der Schmelzanalyse an ein Steuersystem geleitet wird, wobei zwischen Einfluss- und Kompensationselementen unterschieden wird, wobei Einflusselemente Elemente sind, welche einen Einfluss auf zumindest eine werkstoffkundliche Eigenschaft eines Endprodukts haben, und Kompensationselemente Elemente sind, die einen Einfluss auf die zumindest eine werkstoffkundliche Eigenschaft haben und den Einfluss der Einflusselemente kompensieren, wobei die Größe des Einflusses des zumindest einen Ist-Werts der jeweiligen Einfluss- und Kompensationselemente auf die zumindest eine werkstoffkundliche Eigenschaft des Endprodukts bekannt ist, wobei der zumindest eine Ist-Wert bezüglich derjenigen Einflussund Kompensationselemente erfasst wird, welche einen Einfluss auf die zumindest eine werkstoffkundliche Eigenschaft des Endprodukts haben, wobei zur zumindest einen werkstoffkundlichen Eigenschaft des Endprodukts zumindest ein Kompensationselement ermittelt wird, welches die zumindest eine werkstoffkundliche Eigenschaft einstellt, wobei eines oder mehrere Kompensationselemente in die Schmelze in einer Menge zulegiert werden, welche die zumindest eine werkstoffkundliche Eigenschaft in einen Zielbereich bringt und den Einfluss eines oder mehrerer Einflusselemente auf die zumindest eine werkstoffkundliche Eigenschaft kompensiert, und wobei hierfür im Steuersystem zwei Komponenten verwendet werden: ein Prognosemodell und ein Korrekturmodell, wobei mit dem Prognosemodell der Einfluss der Einfluss- und Kompensationselemente auf die zumindest eine werkstoffkundliche Eigenschaft des Endprodukts und mit dem Korrekturmodell korrigierte Zielwerte der Kompensationselemente berechnet werden, um die zumindest eine werkstoffkundliche Eigenschaft in einen Zielbereich zu bringen, sowie ein Steuersystem zur Durchführung des Verfahrens und einen metallischen Hohlkörper, insbesondere ein nahtloses Stahlrohr für Ölfeldanwendungen.

## Beschreibung

Die Erfindung betrifft ein Verfahren zur Herstellung von Vergütungsstählen für Ölfeldanwendungen, ein System zur Herstellung von Vergütungsstählen und einen metallischen Hohlkörper, insbesondere ein nahtloses Stahlrohr für Ölfeldanwendungen.

Weltweit wird Stahl zu 71,1% (in 2023) über die Hochofen-Route erzeugt. Dabei wird Roheisen aus Eisenerz gewonnen, indem dieses zusammen mit Reduktionsmitteln wie bspw. Koks und Zuschlagstoffen im Hochofen geschmolzen wird. Das geschmolzene Roheisen wird anschließend im Konverterverfahren (z. B. mit Sauerstoff) oder im Elektrolichtbogenofen (mit Schrott als Rohstoff) zu Rohstahl weiterverarbeitet.

Primärmetallurgische Aggregate, welche bei der Herstellung von Rohstahl Anwendung finden, können die folgenden sein: BF (Hochofen, Blast Furnace), RE-Ent-S (Roheisenentschwefelung, Hot Metal Desulfurization), BOF (Sauerstoffblaskonverter, Basic Oxygen Furnace), EAF (Elektrolichtbogenofen, Electric Arc Furnace), SAF (Elektroschmelzofen, Submerged Arc Furnace), IF (Induktionsofen, Induction Furnace), Smelter (Schmelzofen), MOE (Schmelzflusselektrolyse, Metal Oxide Electrolysis), Plasmet (Plasmareduktionsmetallurgie) usw. Anschließend wird der Rohstahl in der Sekundärmetallurgie weiterbehandelt, um die Zusammensetzung und Reinheit zu verbessern. Hierbei wird der Stahl in sekundärmetallurgischen Aggregaten wie Spülstand (Rinse Station, Purging Station), Pfannenofen (Ladle Furnace), RH-Anlagen (Ruhrstahl-Heraeus-Verfahren), VD-Anlagen (Vacuum Degassing), DETEM-Anlagen, VOD-Anlagen (Vacuum Oxygen Degassing), ASEA-SKF (Sauerstoff-Vakuumbehandlung mit Rühren), CAS-Anlagen (Composition Adjustment by Sealed Argon Bubbling), RS-V-Anlagen (Rheinstahl-Siemens-Vakuum), CAS-OB-Anlagen (Composition Adjustment by Sealed Argon Bubbling - Oxygen Blowing), Kalk-Silizium Behandlung (CaSi-Injection), Magnesium-Behandlung (Magnesium Injection), VLD-Verfahren (Vacuum Ladle Degassing) etc. behandelt, wo Zusätze (metallische sowie nicht metallische Einsatzstoffe) wie Legierungselemente und Schlackenbilder hinzugefügt werden, um die gewünschte chemische Zusammensetzung und spezifische Eigenschaften des Stahls zu erzielen.

Im Hochofen wird normalerweise kein Schrott eingesetzt, da der Prozess primär auf der Reduktion von Eisenerz basiert, um flüssiges Roheisen zu erzeugen. Schrott wird allerdings in der Stahlproduktion in anderen Prozessschritten genutzt, vor allem in Elektrolichtbogenöfen oder als Kühlmittel in Konvertern.

Das in primär metallurgischen Aggregaten erzeugte Roheisen wird anschließend in einem Sauerstoffkonverter weiterverarbeitet. In diesen Konvertern, wie dem LD-Konverter (Linz-Donawitz-Verfahren), wird flüssiges Roheisen durch Einblasen von Sauerstoff entkohlt und in Rohstahl umgewandelt. Dabei wird Schrott als Kühlmittel und zusätzliche Einsatzstoffe zugegeben. Die Zugabe von Schrott im Konverterprozess hilft, die Temperatur zu kontrollieren, da die Exothermie der Sauerstoffreaktionen die Schmelze stark erhitzt.

Ein anderer wichtiger Produktionsweg in der Stahlindustrie ist der Elektrolichtbogenofen (EAF). Hier kann bis zu 100% Schrott als Einsatzstoff verwendet werden, der durch elektrische Energie eingeschmolzen wird, um flüssigen Stahl herzustellen. Dieses Verfahren ist besonders umweltfreundlich, da zumeist die energieintensivere Reduktion nicht erforderlich ist und daher weniger CO₂-Emissionen verursacht wird als beim Hochofenprozess.

Beim Elektrolichtbogenofenverfahren wird Stahl durch elektrische Energie erzeugt. Dabei wird ein elektrischer Lichtbogen zwischen Graphitelektroden und dem Schrottmaterial im Ofen erzeugt, wodurch hohe Temperaturen (bis zu 3.500 °C im Bereich des Lichtbogens) entstehen, die den Schrott schmelzen und ihn so zu flüssigem Rohstahl verarbeiten.

Bei der Herstellung von Rohstahl werden Einsatzstoffe, wie beispielsweise Schrott, verwendet. Es wird zwischen metallischen und nichtmetallischen Einsatzstoffen unterschieden. Dies sind zum Beispiel Eisenerz, Reduktionsmittel, Schlackenbildner, Eisenschwamm in Form von direkt reduziertem Eisen DRI bzw. heiß brikettiertem Eisen HBI, Desoxidationsmittel, Legierungsmittel und Schrott.

Die Verwendung von Schrott als Rohmaterial für Stahl bietet mehrere Vorteile. Das Einschmelzen von Schrott benötigt lediglich die Energie zum Erwärmen bzw. Schmelzen und, da zumeist die energieintensive Reduktion von vorgelagerten Prozessen durchgeführt wird, weniger Energie als die Primärproduktion von Rohstahl aus Eisenerz. Außerdem reduziert Schrottrecycling die CO₂-Emissionen und den Bedarf an neuen Rohstoffen, was die Umweltbelastung senkt. Da Stahl immer wieder recycelt werden kann, ist die Nutzung von Schrott ein wichtiger Beitrag zur Kreislaufwirtschaft in der Stahlindustrie.

Obwohl die Schrottverwendung im Elektrolichtbogenofen (EAF) viele Vorteile mit sich bringt, gibt es auch einige Nachteile und Herausforderungen, die berücksichtigt werden müssen.

Die erste Herausforderung bei Verwendung von Schrott ist die schwankende Schrottqualität. Der Schrott, der im EAF verwendet wird, ist oft inhomogen und kann Verunreinigungen enthalten, die sich negativ auf die Stahlqualität auswirken. Zum Beispiel können unerwünschte Elemente wie Kupfer, Molybdän, Zinn, Nickel und Chrom im Schrott vorkommen, die im Rohstahl schwer zu entfernen sind und für bestimmte Anwendungen negative Auswirkungen in der Sekundärmetallurgie nachgeschalteten Anlagen und Eigenschaften des Endproduktes haben. Verunreinigungen können den EAF-Prozess komplizierter und teurer machen, da zusätzliche Aufbereitungsschritte oder Sortierungen erforderlich sein können.

Die zweite große Herausforderung betrifft die Kontrolle der chemischen Legierungszusammensetzung. Da Schrott unterschiedliche Legierungen und Metallzusammensetzungen haben kann, kann es schwer sein, die chemische Zusammensetzung des Endprodukts präzise zu steuern. Die Zugabe von Legierungselementen im EAF wird daher komplizierter, und es sind oft zusätzliche Anpassungen nötig, um die gewünschte Stahlspezifikation zu erreichen.

Aufgrund der möglichen Verunreinigungen und der schwankenden Qualität des Schrotts ist es schwer, mit dem EAF-Verfahren besonders niedrige Gehalte (< 0,005 Gew.-%) an Elementen wie Kupfer, Zinn und Zink zu gewährleisten, die für bestimmte spezialisierte Anwendungen erforderlich sind. In solchen Fällen ist die Nutzung von Primärmaterialien (bspw. Roheisen, pig iron, flüssiges Roheisen, hot metal, Eisenschwamm, DRI, HBI) oder sehr hochwertigem, sortenreinem Schrott notwendig, was die Kosten erhöht.

Neben der chemischen Zusammensetzung, welche in einem Elektrolichtbogenofenprozess einsatzstoffbedingten Schwankungen unterliegt und deshalb oft in vorbestimmten Toleranzgrenzen gehalten wird, sind auch die Auswirkungen einzelner Elemente auf die Eigenschaften des Endprodukts zu berücksichtigen.

Insbesondere bei einer nachfolgenden Wärmebehandlung oder Umformprozessen, wie beispielsweise Walzen, werden die werkstoffkundlichen Eigenschaften des Stahls zusätzlich gesteuert.

Es sind bereits einige Lösungen bekannt, welche die unerwünschten Begleitelemente in der Legierungszusammensetzung berücksichtigen oder zusätzliche Anpassungen der Legierungselemente vorsehen.

Es ist üblich, fest definierte Zielbereiche für eine Stahlgüte festzulegen, welche den minimalen und den maximalen Wert je Legierungselement vorsehen. Für Begleitelemente, welche nicht bewusst legiert aber in den Einsatzstoffen in unbekanntem Gehalt enthalten sind, existieren Maximalgehalte, welche helfen, bei Unterschreitung dieser Gehalte die Werkstoffeigenschaften innerhalb des gewünschten Bereiches zu halten. Auf den tatsächlichen Gehalt dieser Begleitelemente wird in der Flüssigphase allerdings nicht reagiert.

Dabei ist von Nachteil, dass niedrige Maximalgehalte der Begleitelemente die Verwendung von Einsatzstoffen mit geringen Gehalten dieser Elemente erzwingen. Dies wird in erster Linie durch einen hohen Anteil an aus Primärstoffen erzeugten Einsatzstoffen erreicht.

Aus der EP 3 956 481 B1 ist ein computergestütztes Verfahren zur Überwachung des Stahlherstellungsprozesses in einem Konverter bekannt. Dabei werden verschiedene Materialien mit spezifischen Eigenschaften in den Konverter eingebracht, um flüssigen Rohstahl und Schlacke zu produzieren. Bei dem Verfahren werden zuerst die gewünschten Eigenschaften des zu produzierenden flüssigen Rohstahls und der Schlacke festgelegt. Dann werden die erforderlichen Mengen jedes Materials, die in den Konverter eingebracht werden müssen, berechnet, um die definierten Zielmerkmale des Rohstahls und der Schlacke zu erreichen. Die berechneten Materialmengen werden an den Bediener oder an automatische Ladesysteme übermittelt und der Konverter wird entsprechend beladen.

Somit wird hier das Beladen des Konverters mit verschiedenen Ausgangsstoffen beschrieben. Nachteilig ist, dass dabei lediglich die Zusammensetzung der im Konverter erzeugten Schmelze kontrolliert wird. Eine nachträgliche Einflussnahme auf die Legierungszusammensetzung wird nicht spezifiziert.

Aus der DE 10 2021 211 320 A1 ist ein Verfahren zur Steuerung und Regelung einer Produktionsanlage für Walzprodukte aus metallischen Legierungen wie Stahl, Eisen oder Aluminium bekannt. Ziel des Verfahrens ist es, den Einsatz von Rohstoffen zu optimieren und die Produktionskosten zu senken. Anfangs werden für jeden Auftrag spezifische Sollwerte für Material-, Oberflächen- und geometrische Eigenschaften des Endprodukts sowie zulässige chemische Zusammensetzungen mit definierten Toleranzbereichen festgelegt. Durch Anwendung von Prozessmodellen werden für jeden Auftrag prädiktive Ist-Werte der Produkteigenschaften berechnet und mit den Sollwerten verglichen. Nur Zusammensetzungen, die innerhalb der spezifizierten Toleranzen liegen, werden ausgewählt. Für jede ausgewählte chemische Zusammensetzung werden Kostenparameter wie Legierungs-, Einsatzstoff-, Energie-, Eisen-, Zuschlagsstoff- und CO₂-Kosten ermittelt. Diese werden in Form von Strafpunkten oder einer Straffunktion quantifiziert. Anschließend wird die Schnittmenge der zulässigen chemischen Zusammensetzungen für verschiedene Kombinationen von Produktionsaufträgen, die in einer gemeinsamen Charge erschmolzen werden sollen, ermittelt.

Nachteilig dabei ist, dass hier Toleranzbereiche für jede Zusammensetzung ermittelt werden. Eine genaue Steuerung der Zusammensetzung ist mit diesem Modell nicht möglich.

Aus der EP 4 183 498 A1 ist ein KI-basiertes Prognosemodell bekannt, mit welchem sich die mechanischen Eigenschaften aus einigen vorher gemessenen Ausgangswerten, wie beispielsweise der Zusammensetzung der Schmelze, vorhersagen lassen. Um einen Einfluss auf die mechanischen Eigenschaften zu nehmen, wird als Output eine Anpassung der Herstellungsparameter, wie beispielsweise Vorwalzverhältnis, Fertigwalzverhältnis, Walzstarttemperatur, Startzeit der Kühlung, Abkühlungsrate oder Liniengeschwindigkeit vorgeschlagen.

Das Modell ist auf spezifische Produktionslinien und -prozesse optimiert. Wenn die Produktionsbedingungen oder Anforderungen schnell geändert werden müssen, kann das System Schwierigkeiten haben, sich anzupassen.

Vergütungsstahl ist Stahl, welcher durch Vergüten, d.h. durch Härten und anschließendes Anlassen, hergestellt wird und hohe Zug- und Dauerfestigkeit aufweist. Die Härte des Vergütungsstahls wird dabei durch das Material (chemische Zusammensetzung) und die Prozessparameter während des Härtens (gewählte Härtetemperatur und Abschreckgeschwindigkeit) bestimmt. Durch die vorgenommene Umwandlungshärtung kann das Verhältnis von Festigkeit zu Zähigkeit gezielt beeinflusst werden.

Vergütungsstahl findet Anwendung bei der Herstellung von Kurbelwellen, Wellen, Achsen, Bolzen, Schrauben und anderer Konstruktionsteile hoher Festigkeit, wie zum Beispiel nahtloser Stahlrohre zum Einsatz in der Öl- und Gasindustrie.

Die Vergütungsstähle für nahtlose Stahlrohre zum Einsatz in der Öl- und Gasindustrie basieren auf den folgenden Normen:
- API Specification 5CT: "Casing and tubing" (dt. Gehäuse und Rohre)
- API Specification 5L: "Line pipe" (dt. Rohrleitung)
- EN ISO 11960: "Erdöl- und Erdgasindustrie - Stahlrohre zur Verwendung als Futter- oder Steigrohre für Bohrungen"

Die Vergütungsstähle in den Festigkeitsklassen bis einschließlich 80 ksi (kilopound per square inch), entsprechend 552 MPa, werden als naturharte bzw. normalisierte Rohre produziert. Die Festigkeitsklassen ab 80 ksi werden mittels eines spezifischen Vergütungsprozesses (Härten, Wasserquenchen und Anlassen) dargestellt. Neben den Parametern aus der Prozessführung, wie zum Beispiel Temperatur und Zeit, ist hierbei die chemische Zusammensetzung des Stahls maßgeblich für die Ausbildung der Mikrostruktur und damit der Festigkeit.

Auch die Korrosionsbeständigkeit des Materials ist in der Anwendung eine wesentliche Produkteigenschaft. Für den konkreten Anwendungsfall ist besonders die Beständigkeit gegenüber Sauergas (H₂S) von Bedeutung. Die für Vergütungsstähle für Ölfeldanwendungen erforderliche Korrosionsbeständigkeit ist wiederum von einer ausgewogenen und abgestimmten Legierungszusammensetzung und einer homogenen Mikrostruktur abhängig.

Die Hauptlegierungselemente in solchen Vergütungsstählen sind Kohlenstoff, Mangan, Silizium, Molybdän und Chrom. Daneben gilt es Begleitelemente wie Schwefel, Phosphor und Kupfer entsprechend einzuschränken.

Die werkstoffkundlichen, insbesondere mechanischen Eigenschaften von Vergütungsstählen lassen sich durch eine gezielte Zugabe von Legierungselementen steuern. Die exakte Legierungszusammensetzung ist allerdings im EAF-Verfahren aufgrund der durch die Einsatzstoffe, beispielsweise Schrott, eingebrachten Begleitelemente nur schwer zu kontrollieren. Es müssen jedoch auch im EAF-Verfahren Methoden gefunden werden, um Zielvorgaben der jeweiligen Vergütungsstähle hinsichtlich ihrer werkstoffkundlichen, insbesondere mechanischen Eigenschaften zu erreichen.

Aufgabe der Erfindung ist es, ein Verfahren zur Herstellung von Vergütungsstählen für Ölfeldanwendungen zur Verfügung zu stellen, welches eine gezielte Steuerung der werkstoffkundlichen, insbesondere mechanischen Eigenschaften auch bei größeren Schwankungen der Begleitelemente ermöglicht.

Die Aufgabe wird mit einem Verfahren mit den Merkmalen des Anspruchs 1 gelöst.

Vorteilhafte Weiterbildungen sind in den Unteransprüchen gekennzeichnet.

Ferner ist es Aufgabe der Erfindung, ein System zur Herstellung von Vergütungsstählen zur Verfügung zu stellen, welches eine gezielte Steuerung der werkstoffkundlichen, insbesondere mechanischen Eigenschaften auch bei größeren Schwankungen der Begleitelemente ermöglicht.

Diese Aufgabe wird mit einem Steuersystem mit den Merkmalen des Anspruchs 18 gelöst.

Vorteilhafte Weiterbildungen sind in den Unteransprüchen gekennzeichnet.

Ferner ist es auch Aufgabe der Erfindung, einen metallischen Hohlkörper, insbesondere ein nahtloses Stahlrohr für Ölfeldanwendungen aus einem Vergütungsstahl zur Verfügung zu stellen.

Diese Aufgabe wird mit einem metallischen Hohlkörper mit den Merkmalen des Anspruchs 23 gelöst.

Es handelt sich um ein erfindungsgemäßes Konzept eines dynamischen Legierens. Dabei wird eine Adaption der Elementgrenzen für Legierungselemente in der Flüssigphase durchgeführt.

Durch eine Analyse der Schmelze werden Ist-Werte der Elementgehalte bestimmt. Diese Ist-Werte werden in ein für einen spezifizierten Vergütungsstahl passendes, datengetriebenes Prognosemodell eingespeist, sodass der Einfluss der jeweiligen Elemente auf eine werkstoffkundliche, insbesondere mechanische Eigenschaft modelliert und, wenn dies notwendig ist, um die werkstoffkundliche, insbesondere mechanische Eigenschaft in einen gewünschten Zielbereich zu bringen, durch ein Korrekturmodell eine Reaktion in Form von adaptierten Legierungselementgrenzen aufgefunden wird und diese dem Legierungsrechner als adaptierte Zielwerte zugeführt werden. Der Legierungsrechner benutzt die adaptierten Legierungselementgrenzen zur Festlegung der Massen an Einsatzstoffen.

Die Erfinder haben erkannt, dass bei der dynamischen Korrektur der Elementgehalte zwischen zwei definierten Elementarten unterschieden werden soll. Es handelt sich hierbei um Einfluss- und Kompensationselemente.

Bei der Stahlherstellung werden beim Herstellungsprozess verschiedene Einsatzstoffe verwendet. Dies sind zum Beispiel Eisenerz, Eisenschwamm in Form von direkt reduziertem Eisen (DRI) bzw. heiß brikettiertem Eisen (HBI) und Schrott. Über diese Einsatzstoffe wird natürlich nicht nur Eisen eingebracht, sondern auch sogenannte Begleitelemente und Spurenelemente.

Begleitelemente sind üblicherweise nicht vermeidbare Elemente, wie beispielsweise Si, Mn, S, P, O, N und H, und werden im Verlauf eines nachfolgenden metallurgischen Prozesses entfernt, sofern das möglich ist. Ist ein Entfernen nicht möglich, müssen das jeweilige Begleitelement und sein Einfluss bei metallurgischen Prozessen berücksichtigt werden.

Für jede Stahlsorte, welche durch die chemische Zusammensetzung der Legierung und die Herstellroute definiert wird, existiert eine definierte, aus praktischen Erfahrungswerten resultierende Begleitelementklasse, d.h. es müssen bestimmte Obergrenzen der Gehalte der Begleitelemente eingehalten werden.

Bei Spurenelementen handelt es sich um explizit störende Begleitelemente (z.B. As und Sb), welche in sehr geringen Mengen vorliegen. Diese werden im Zuge des (hier durchgeführten dynamischen) Legierens nicht berücksichtigt.

Legierungselemente sind Elemente, welche gezielt zugesetzt werden, um dem Stahl gewünschte, werkstoffkundliche Eigenschaften zu verleihen. Solche Eigenschaften können mechanische Eigenschaften wie Härte, Zugfestigkeit, Zähigkeit und weitere Eigenschaften sein, aber auch chemische Eigenschaften wie Widerstand gegen Korrosion oder Wasserstoffversprödung und weitere.

Bei Einsatz von Einsatzstoffen, insbesondere metallischen Einsatzstoffen und nicht metallischen Einsatzstoffen, muss beachtet werden, dass sie für sich gesehen Begleitelemente aus ihrer Rohstoffhistorie und Legierungselemente aus ihrer metallurgischen Historie enthalten.

Wird nun beispielsweise Schrott als Einsatzstoff in der Stahlherstellung verwendet, sind seine Bestandteile, bis auf Eisen, für das Produkt, was unter Einsatz des Schrotts erzeugt werden soll, Begleit- und Spurenelemente.

Deren Anwesenheit muss also berücksichtigt werden, da sie (als ehemalige Legierungselemente) aus dem Produkt über metallurgische Prozesse entweder nur mit großem Aufwand oder nicht entfernbar sind. Jedoch kann ihre Wirkung erfindungsgemäß berücksichtigt und kompensiert werden.

Im klassischen Herstellungsprozess wird von einem Produkt (Stahl) ausgegangen, welches entsprechend den Marktanforderungen bestimmte werkstoffkundliche Eigenschaften aufweist. Auf dieses Anforderungsprofil hin, wird es chemisch/metallurgisch eingestellt. Ein solches Produkt ist also eine Legierung, wobei die Legierungsbestandteile durch einen definierten Gehalt an Obergrenzen oder mit Unter- und Obergrenzen bestimmt sind. Von einer bestimmten Legierung ist somit bekannt, welche Eigenschaften sie haben kann.

Wird nun aufgrund geänderter Bedingungen, der Einsatz von Einsatzstoffen wie bspw. Schrott erheblich erhöht, wird das Produkt folgerichtig mit einer höheren Menge von Begleitelementen befrachtet. Diese Begleitelemente haben (als "ehemalige" Legierungselemente) einen Einfluss zum Beispiel auf werkstoffkundliche Eigenschaften des Produkts.

Insofern muss dies bei dem Zulegieren berücksichtigt werden. Hierbei können die folgenden Fälle eintreten.
A) Beispielsweise wird für Element A durch den Einsatzstoff ein Anteil eingebracht, der unterhalb des gewünschten Gehalts liegt. In diesem Fall muss ggf. weniger Element A zulegiert werden.
B) Für Element A wird über den Einsatzstoff ein Gehalt eingebracht, der bereits im Zielfenster liegt, es muss nichts mehr zulegiert werden.
C) Es wird mehr Element A als gewünscht eingebracht. Der Gehalt des Elements A kann nur unter erhöhtem Aufwand metallurgisch verringert werden. Dementsprechend muss die Wirkung des Elements A kompensiert werden.

Das obige Beispiel wird dann komplexer, wenn mehrere Begleitelemente oder Legierungselemente für bestimmte Eigenschaften zusammenspielen.

Hier setzt die Erfindung dadurch an, dass zu den Begleitelementen, die einen Einfluss auf eine werkstoffkundliche, insbesondere mechanische Eigenschaft des Vergütungsstahls haben (nachfolgend Einflusselemente genannt), Legierungselemente ausgewählt werden, die den Einfluss des Einflusselements auf die werkstoffkundliche, insbesondere mechanische Eigenschaft kompensieren können (nachfolgend Kompensationselemente genannt).

Kompensationselemente sind Elemente, die bestenfalls ohnehin reguläre Legierungselemente der Ziellegierung sind, sodass im Rahmen der qualitativen gewünschten Legierungszusammensetzung lediglich quantitative Anpassungen erfolgen.

Ein einfaches Beispiel soll dies deutlich machen. Sind neben Eisen in einer gewünschten Stahllegierung die Elemente A, B und C enthalten und ist das Element A in einer Menge vorhanden, die die gewünschte Menge übersteigt, und ist C ein Element, welches den Einfluss von A auf eine bestimmte werkstoffkundliche, insbesondere mechanische Eigenschaft verringert, wird C in einer Menge zulegiert, welche zusätzlich zur eigenen Wirkung noch die Wirkung von A kompensiert.

Ein weiteres Beispiel ist, wenn Element A durch die Einsatzstoffe in einer Menge vorhanden ist, die die gewünschte Menge überschreitet. Wenn das Element C einen vergleichbaren Einfluss auf die werkstoffkundliche, insbesondere mechanische Eigenschaft hat wie A, jedoch in der Legierung wenig oder nur in Spuren vorhanden ist, wird hiervon nur so viel zugesetzt, dass die addierte Wirkung von A und C gemeinsam die durch die erwünschte Vergütungsstahlgüte spezifizierte werkstoffkundliche, insbesondere mechanische Eigenschaft zur Folge haben. D.h. in diesem Fall, dass weniger C zugesetzt wird als normalerweise zugesetzt würde, um den zu hohen A-Gehalt zu kompensieren.

Kompensation im Sinne der Erfindung bedeutet somit, dass eine Anpassung der Ziellegierungszusammensetzung nach oben oder unten stattfinden kann.

B ist in diesem Fall z.B. ein Element, welches auf die werkstoffkundliche, insbesondere mechanische Eigenschaft keinen Einfluss hat.

Insgesamt kann durch dieses erfindungsgemäße Vorgehen, die Einsatzstoffvorauswahl gröber erfolgen.

Eingangskenngrößen in das Prognosemodell sind alle Elemente, die in der Flüssigphase enthalten und analysiert worden sind.

Ausgangsgrößen sind adaptierte Elementgrenzen für Elemente, welche in der Flüssigphase legiert werden. Es handelt sich hierbei um die sogenannten Kompensationselemente, wie beispielsweise C, Si, Mn, Cr. Kompensationselemente sind somit Elemente, welche in der Flüssigphase zulegiert werden, um den Einfluss der Einflusselemente auszugleichen, damit die Soll-Eigenschaften der Endlegierung erreicht werden.

Sowohl die Einfluss- als auch die Kompensationselemente sind Elemente, welche durch Zugabe von Einsatzstoffen in die Schmelze eingebracht werden.

Dabei handelt es sich um Begleitelemente, die im Eisen oder Stahl unvermeidlich vorkommen und in geringer Menge enthalten sein können. Sie können aus den verwendeten Rohstoffen, wie Erz oder Schrott, stammen oder bei der Verarbeitung in die Schmelze eingebracht werden. Diese Begleitelemente beeinflussen die Eigenschaften des Stahls und können je nach Gehalt positive oder negative Effekte auf ausgewählte werkstoffkundliche, insbesondere mechanische Eigenschaften haben.

In der Stahlherstellung wird "Schrott" als recycelter, wiederverwendbarer metallischer Sekundärstoff bezeichnet, der als Einsatzstoff zur Produktion von neuem Stahl dient. Schrott besteht hauptsächlich aus Eisenbasislegierung und wird in verschiedenen Qualitäten und Sorten klassifiziert, je nach Reinheit, Form, Größe und chemischer Zusammensetzung.

Das vorliegende Verfahren, bei dem Einsatzstoffe eingesetzt werden, betrifft die Herstellung von Vergütungsstählen für Ölfeldanwendungen mit auf eine Vergütungsstahlgüte spezifizierten werkstoffkundlichen, insbesondere mechanischen Eigenschaften.

Das Konzept beruht darauf, ein Endprodukt einer bestimmten Vergütungsstahlgüte zu erzeugen, bei dem sich eine werkstoffkundliche, insbesondere mechanische Eigenschaft trotzt erhöhter Einflusselementgehalte, welche durch die Einsatzstoffen in metallurgischen Aggregaten eingebracht werden, gar nicht oder nur möglichst geringfügig ändert und zwar im Vergleich zu der werkstoffkundlichen, insbesondere mechanischen Eigenschaft, die sich einstellt, wenn bei der Herstellung der Schmelzen Einsatzstoffe mit geringen Anteilen an Einflusselementen eingesetzt werden und damit geringere Anteile an unerwünschten Begleitelementen eingebracht werden (z.B. Primärroute), so dass das Produkt innerhalb eines durch die Vergütungsstahlgüte spezifizierten Zielbereichs hinsichtlich der werkstoffkundlichen, insbesondere mechanischen Eigenschaft liegt. Dies stellt auch sicher, dass auf die Stahlherstellung folgende Nachbehandlungsschritte nicht geändert werden müssen.

Dies geschieht durch Adaption eines oder mehrerer Legierungselemente, und wird nachfolgend als dynamisches Legieren bezeichnet. Die Adaption erfolgt durch Anwendung eines Prognosemodells, welches zunächst aufgrund von Analyse-Ist-Werten die erwartbare werkstoffkundliche, insbesondere mechanische Eigenschaft modelliert. Sollte diese außerhalb des durch die gewünschte Vergütungsstahlgüte spezifizierten Zielbereichs liegen, werden mittels eines Korrekturmodells korrigierte Legierungselementgehalte berechnet, sodass die spezifizierten Anforderungen an die werkstoffkundliche, insbesondere mechanische Eigenschaft erfüllt werden können.

Für Vergütungsstähle relevante mechanische Eigenschaften sind dabei beispielsweise die Zugfestigkeit (tensile strength - TS), die Dehngrenze (yield strength - YS), die Zähigkeit, die Streckgrenze und die Korrosionsbeständigkeit. Besonders die Zugfestigkeit und die Dehngrenze soll durch das erfindungsgemäße Verfahren steuerbar werden. Zudem sind auch Härtbarkeitsindizes, wie zum Beispiel das Kohlenstoffäquivalent CAE, als Kenngrößen von Bedeutung und sollen durch das erfindungsgemäße Verfahren berücksichtigt werden.

Für die Erzeugung des Prognose- und/oder Korrekturmodells können unterschiedliche Machine-Learning-Modelle herangezogen werden, beispielsweise lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

Diese Modelle sind datengetrieben. Als Datenbasis für die chemischen und mechanischen Werte können beispielsweise datenbankbasierte Abfragen herangezogen werden. Auch Prozessparameter, insbesondere Wärmebehandlungstemperatur und Haltzeiten, aus entsprechenden datenbankbasierten Abfragen können genutzt werden. Ferner können Datensätze auch durch Simulationsdaten erweitert werden, um den Einfluss höherer Einflusselementfracht abzubilden. Es findet eine Aufteilung der Datenbasis in Trainings- und Validierungsdaten statt, beispielsweise werden 80% der Daten zu Trainingszwecken und 20% der Daten zu Validierungszwecken verwendet.

Zur Verbesserung der Genauigkeit der Modelle könnten zusätzlich zur chemischen Zusammensetzung weitere wichtige Prozessparameter, wie beispielsweise die Temperaturführung des Walz- und Abkühlprozesses, in den Modellen berücksichtigt werden. Zudem ist es hierfür sinnvoll hinsichtlich einer bestimmten werkstoffkundlichen, insbesondere mechanischen Eigenschaft die relevantesten Wirkparameter und gegebenenfalls vernachlässigbare Parameter zu identifizieren.

Rein datengetriebene Modelle eignen sich gut für eine Vorhersage im durch die Trainingsdaten bestimmten Datenraum. Die Vorhersage mag sich jedoch verschlechtern, sollte signifikant von diesem bekannten Datenraum abgewichen werden, d.h. wenn eine stark abweichende Legierungszusammensetzung in die Berechnung der werkstoffkundlichen, insbesondere mechanischen Eigenschaft mittels des Prognose- und/oder Korrekturmodells einfließt. Kleine Trainingsdatensätze leiden zudem gegebenenfalls unter großer Streuung, sodass die Vorhersagegenauigkeit beeinträchtigt werden kann. Dieser Effekt kann durch größere Trainingsdatensätze minimiert werden.

Um für die Modellierung der werkstoffkundlichen, insbesondere mechanischen Eigenschaft das Modell mit der höchsten Genauigkeit zu identifizieren, werden die durch Analyse der Schmelze bestimmten Ist-Werte in mögliche Modelle (siehe obige Aufzählung) eingespeist. Das Prognosemodell und/oder Korrekturmodell mit der höchsten Genauigkeit, welches sich durch die geringste Abweichung/den geringsten Wert der Quadratwurzel des mittleren quadratischen Fehlers (root mean square error, RMSE) auszeichnet, wird schließlich zur Modellierung der werkstoffkundlichen, insbesondere mechanischen Eigenschaft herangezogen. Bei Betrachtung mehrerer werkstoffkundlicher, insbesondere mechanischer Eigenschaften wird der Mittelwert und/oder die Summe der Quadratwurzeln der mittleren quadratischen Fehler der werkstoffkundlichen, insbesondere mechanischen Eigenschaften herangezogen.

Die Zugfestigkeit TS und die Dehngrenze YS können in einem auf multipler linearer Regressionsanalyse basierenden Prognose- und/oder Korrekturmodell gemäß den folgenden Formeln berechnet werden: $TS = {α}_{0} + {α}_{1} \times {X}_{1} + α \times {X}_{2} + ⋯ + {α}_{n} \times {X}_{n} ;$ $YS = {β}_{0} + {β}_{1} \times {X}_{1} + {β}_{2} \times {X}_{2} + ⋯ + {β}_{n} \times {X}_{n} ,$ wobei
- α₀ und β₀: Basisfaktoren der Zugfestigkeit TS und Dehngrenze YS,
- α₁- αₙ und β₁- βₙ: Regressionskoeffizienten und
- X₁-Xₙ: Elementgehalte in Gew.-% sind.

Die berechneten Werte für die werkstoffkundlichen, insbesondere mechanischen Eigenschaften werden mit ihren jeweiligen Zielwerten verglichen, um die Notwendigkeit des dynamischen Legierens zu beurteilen, welches nur nötig wird, wenn die werkstoffkundliche, insbesondere mechanische Eigenschaft außerhalb eines definierten Zielbereichs liegt.

Neben den werkstoffkundlichen, insbesondere mechanischen Eigenschaften sind sog. Härtbarkeitsindizes, welche ein Maß für die Härtbarkeit des Stahls darstellen, wichtig. Als Härtbarkeitsindex können eine Vielzahl von bekannten Parametern herangezogen werden. Beispielsweise, jedoch nicht ausschließlich, sind Härtbarkeitsindizes wie in den folgenden Normen oder Patentschriften beschrieben möglich: DIN EN 10025-2, EN 1011-2, ASTM A255 DE69607702T2, EP2692890, EP2695960B1, EP2589676B1, EP3561130B1, EP2726637B2, EP2562272B1, DE102015221387A1, EP2258880B1, EP2514844B1. Derartige Parameter können beispielsweise auch den Veröffentlichungen "Hardness Prediction in Quenched and Tempered Nodullar Cast Iron Using the Hollomon-Jaffe Parameter" (Guillanön et al., Metals 2021, 11(2), 297) und "A historical overview of steel tempering parameters" (Totten et al., Int. J. Microstructure and Materials Properties, Vol. 3, Nos. 4/5, 2008) entnommen werden.

Das Kohlenstoffäquivalent wird häufig als ein solcher Härtbarkeitsindex herangezogen. Grundsätzlich ist das Kohlenstoffäquivalent ein Maß für die Schweißbarkeit eines Stahls und dient zur Vermeidung von Härterissen und zur Abschätzung der Notwendigkeit des Vorwärmens. Zudem ist das Kohlenstoffäquivalent geeignet, um den Einfluss von Elementen auf die Härtbarkeit und Verarbeitbarkeit im Vergütungsprozess zu beurteilen. Das Kohlenstoffäquivalent kann als beschreibend für die Grundgesamtheit einer Stahllegierung herangezogen werden.

Grundsätzlich gibt es eine Vielzahl von Definitionen von Kohlenstoffäquivalenten im Stand der Technik, welche allesamt ohne weiteres bei der Modellierung nach der Erfindung Anwendung finden können.

Die Berechnung des Kohlenstoffäquivalents ist abhängig von der betreffenden Legierung bzw. von den zugrundeliegenden Normen und/oder Spezifikationen. Es kommen beispielsweise, jedoch nicht ausschließlich, folgende Formeln im Prognosemodell zum Einsatz: $\begin{matrix}CEV \left(Carbon Equivalent Value\right) = Gew . - % C + \frac{Gew . - % Mn}{6} + \frac{Gew . - % Cr + Gew . - % Mo + Gew . - % V}{5} + \\ \frac{Gew . - % Ni + Gew . - % Cu}{15} ;\end{matrix}$ $\begin{matrix}CET \left(Carbon Equivalent Thyssen\right) = Gew . - % C + \frac{Gew . - % Mn + Gew . - % Mo}{10} + \frac{Gew . - % Cr + Gew . - % Cu}{20} + \\ \frac{Gew . - % Ni}{40} ;\end{matrix}$ $\begin{matrix}Pcm \left(Critical Metal Parameter\right) = Gew . - % C + \frac{Gew . - % Si}{30} + \frac{Gew . - % Mn + Gew . - % Cu + Gew . - % Cr}{20} + \\ \frac{Gew . - % Mo}{15} + \frac{Gew . - % Ni}{60} + \frac{Gew . - % V}{10} + 5 \times Gew . - %B ,\end{matrix}$ wobei Gew.-%i der Gehalt des jeweiligen Elements i in Gew.-% ist.

Die Modellierung der werkstoffkundlichen, insbesondere mechanischen Eigenschaft zeigt, ob dynamisches Legieren notwendig ist, damit die betreffende Eigenschaft in einen spezifizierten Zielbereich fällt. Liegt die werkstoffkundliche, insbesondere mechanische Eigenschaft im Zielbereich, findet keine Kompensation der Einflusselemente durch ausgewählte Kompensationselemente statt. Weicht die werkstoffkundliche, insbesondere mechanische Eigenschaft jedoch vom vorgesehenen Zielbereich ab, werden durch das Korrekturmodell adaptierte Elementgehalte der Ziellegierung berechnet. Entsprechend wird zumindest ein Kompensationselement zulegiert, um die werkstoffkundliche, insbesondere mechanische Eigenschaft in den Zielbereich zu bringen.

Nach der Zugabe von Kompensationselementen kann eine weitere Analyse getätigt werden. Der Ist-Wert der Legierungszusammensetzung wird kontrolliert und bei Bedarf erneut dynamisch mithilfe des Korrekturmodells korrigiert.

Die Elemente Cu, Mo und Ni werden als Einflusselemente betrachtet. Als Kompensationselemente werden grundsätzlich C, Si, Mn und/oder Cr gewählt.

Hiervon ausgenommen sind zum Beispiel bestimmte legierte Stähle wie folgt: Bei Ni-legierten Vergütungsstählen zählen nur Cu und Mo, bei Mo-legierten Vergütungsstählen nur Cu und Ni und bei Ni-Mo-legierten Vergütungsstählen nur Cu zu den Einflusselementen. In diesen Fällen sind entsprechend die zuvor genannten Legierungselemente auch als Kompensationselemente geeignet.

Der Einfluss der einzelnen Elemente auf die werkstoffkundlichen Eigenschaften der Legierung ist wie folgt.

Kupfer wird als Legierungselement in herkömmlichen Stählen aufgrund der Gefahr von Duktilitätsverlust und interkristallinen Oberflächenrissen nur in äußerst geringen Mengen zugesetzt. Kupfer hat zudem einen negativen Einfluss auf die Kaltverformbarkeit, Warmumformbarkeit und Kerbschlagzähigkeit und vermindert die Schweißneigung. Zu hohe Kupfergehalte können zu unerwünschter Rotbrüchigkeit führen, welche bei der Warmumformung durch Bildung einer Kupferschmelze aufgrund von selektiver Korrosion auftritt und bei geringsten Belastungen zu Rissen oder Brüchen führen kann. Der Rotbrüchigkeit kann z.B. durch Zulegieren von Nickel entgegengewirkt werden. Andererseits kann Kupfer die Streckgrenze, Zugfestigkeit und Härtbarkeit steigern. Durch Ausscheidungshärtung trägt Kupfer effektiv zur Festigkeitssteigerung bei. Bei wetterfesten Stählen kann ein Zulegieren von Kupfer die Witterungs- und Korrosionsbeständigkeit erhöhen.

Es ist vorteilhaft, wenn ein Kupfergehalt von 0,001 bis 0,5 Gew.-%, vorzugsweise 0,001 bis 0,45 Gew.-%, besonders bevorzugt 0,001 bis 0,42 Gew.-%, weiterhin besonders bevorzugt 0,001 bis 0,4 Gew.-% gewählt wird.

Besonders über die EAF-Route erzeugte Stahlschmelzen weisen aufgrund des erhöhten Schrotteinsatzes oft zu hohe Kupfergehalte auf. Die Entfernung von Kupfer aus der Schmelze ist äußerst schwierig, sodass seine Wirkung durch andere Legierungselemente kompensiert werden muss.

Nickel erhöht durch die Verringerung der kritischen Abkühlgeschwindigkeit die Durchhärtung und Durchvergütung. Zudem erhöht es die Zähigkeit, Härte und Festigkeit von Stahl und außerdem die Löslichkeit von Kupfer im Stahl, sodass unerwünschter Rotbrüchigkeit entgegengewirkt wird. Auch die Korrosionsbeständigkeit von Stahl kann durch Nickel verbessert werden. Nachteilig sind jedoch die hohen Kosten, die bei Verwendung von Nickel als Legierungselement entstehen.

Es ist vorteilhaft, wenn ein Nickelgehalt von 0,001 bis 4,0 Gew.-%, vorzugsweise 0,001 bis 3,8 Gew.-%, besonders bevorzugt 0,001 bis 3,5 Gew.-%, weiterhin besonders bevorzugt 0,001 bis 3,0 Gew.-% gewählt wird.

Molybdän wechselwirkt stark mit Phosphor, sodass es dessen schädigende Wirkung im Stahl (Entmischung an den früheren Austenit-Korngrenzen) begrenzt. Durch ausgeprägtes karbidbildendes Verhalten steigert Molybdän die Festigkeit des Stahls, während es jedoch die Duktilität verringert. Molybdänkarbide erhöhen außerdem die Verschleißfestigkeit und Anlassbeständigkeit und können als Wasserstofffallen wirken, wodurch die Beständigkeit gegen verzögerte Brüche verbessert wird. Darüber hinaus wird die Schweißbarkeit des Stahles mit steigendem Molybdängehalt vermindert.

Es ist vorteilhaft, wenn ein Molybdängehalt von 0,001 bis 1,5 Gew.-%, vorzugsweise 0,001 bis 1,45 Gew.-%, besonders bevorzugt 0,001 bis 1,4 Gew.-%, weiterhin besonders bevorzugt 0,001 bis 1,35 Gew.-% gewählt wird.

Zinn wirkt verfestigend und härtesteigernd, vermindert zugleich die Bruchdehnung und neigt zu stärkeren Seigerungen, wodurch die Gefügestabilität beeinflusst wird. Zinn wirkt darüber hinaus oberflächenaktiv und verschlechtert die Warmumformbarkeit.

Es ist vorteilhaft, wenn ein Zinngehalt von 0,001 bis 0,03 Gew.-%, vorzugsweise 0,001 bis 0,028 Gew.-%, besonders bevorzugt 0,001 bis 0,026 Gew.-%, weiterhin besonders bevorzugt 0,001 bis 0,025 Gew.-% gewählt wird.

Als Karbidbildner mit den unterschiedlichsten Elementen (z.B. Zementit, Fe₃C) erhöht Kohlenstoff die Härte, Festigkeit und Streckgrenze des Stahls. Geringe Kohlenstoffgehalte sichern eine gute Umformbarkeit und eine gute Schweißbarkeit, können jedoch keine hohe Festigkeit bedingen. Hohe Kohlenstoffgehalte erhöhen die Festigkeit, was aber auch die Gefahr der Versprödung und damit der Rissbildung birgt. Eine der wichtigsten Eigenschaften des Kohlenstoffs als Legierungselement ist die Möglichkeit, umwandlungsfähigen Stahl durch Abschreckung zu härten.

Es ist vorteilhaft, wenn ein Kohlenstoffgehalt von 0,05 bis 1,2 Gew.-%, vorzugsweise 0,05 bis 1,1 Gew.-%, besonders bevorzugt 0,05 bis 1,05 Gew.-%, weiterhin besonders bevorzugt 0,05 bis 1,0 Gew.-% gewählt wird.

Silizium hat einen günstigen Einfluss auf die Duktilität und die Festigkeit des Stahls. Zudem verbessert es die Zunderbeständigkeit und vermindert so die Gefahr der Rotbrüchigkeit. Aufgrund seiner hohen Sauerstoffaffinität ist es ein wichtiges Desoxidationsmittel. Hohe Siliziumgehalte führen durch Mischkristallbildung zur Verfestigung des Gefüges. Bei zu hohen Gehalten kann es jedoch eine schädigende Wirkung bspw. auf die Kaltumformbarkeit haben. Silizium erhöht die Streckgrenze und die Zugfestigkeit, ohne die Dehnung wesentlich zu verringern. Weiter vermindert das Element die kritische Abkühlgeschwindigkeit und vergrößert damit die Einhärtung. Silizium erhöht auch die Zunderbeständigkeit und hat einen signifikanten Einfluss auf die elektrischen Eigenschaften des Stahls.

Es ist vorteilhaft, wenn ein Siliziumgehalt von 0,001 bis 1,9 Gew.-%, vorzugsweise 0,001 bis 1,85 Gew.-%, besonders bevorzugt 0,001 bis 1,8 Gew.-%, weiterhin besonders bevorzugt 0,001 bis 1,75 Gew.-% gewählt wird.

Mangan hat bei niedrigen Gehalten eine vorteilhafte Wirkung auf das Seigerungsverhalten im Strangguss und verbessert die Umformbarkeit. Bezüglich Härtbarkeit und Durchhärtung wirkt sich Mangan positiv aus. Höhere Mangangehalte bedingen eine höhere Grundfestigkeit, erhöhen aber auch das Risiko der Wasserstoffversprödung, weil Mangan die Entmischung von Phosphor und Schwefel an den früheren Austenitkorngrenzen beschleunigt. Mangan ist ein wirksames Desoxidationsmittel und ein starker Austenitstabilisator. Auch kann es zur Vermeidung von Rotbrüchigkeit beitragen.

Es ist vorteilhaft, wenn ein Mangangehalt von 0,1 bis 3,5 Gew.-%, vorzugsweise 0,1 bis 3,4 Gew.-%, besonders bevorzugt 0,1 bis 3,2 Gew.-%, weiterhin besonders bevorzugt 0,1 bis 3,0 Gew.-% gewählt wird.

Chrom erhöht die Zugfestigkeit, während die Dehnung nur geringfügig verschlechtert wird. Durch Herabsetzen der Bainit-Starttemperatur des Stahls führt Chrom zu einer Verfeinerung der bainitischen Struktur. Zusätzlich wird die Härte sowie die Beständigkeit gegen Wasserstoffbruch durch Karbidbildung erhöht. Höhere Chromgehalte verbessern auch die Warmfestigkeit und Anlassbeständigkeit. Chrom hat jedoch einen negativen Einfluss auf die Schweißbarkeit.

Es ist vorteilhaft, wenn ein Chromgehalt von 0,001 bis 15 Gew.-%, vorzugsweise 0,001 bis 14,8 Gew.-%, besonders bevorzugt 0,001 bis 14,6 Gew.-%, weiterhin besonders bevorzugt 0,001 bis 14,5 Gew.-% gewählt wird.

Vanadium hat eine härtende Wirkung und ermöglicht so das Bainitisieren bei höheren Temperaturen. Zu hohe Vanadiumgehalte führen zu Ausscheidungen, welche die Widerstandsfähigkeit des Stahls gegen verzögerten Wasserstoffbruch beeinträchtigen.

Es ist vorteilhaft, wenn ein Vanadiumgehalt von 0,001 bis 0,4 Gew.-%, vorzugsweise 0,001 bis 0,40 Gew.-%, besonders bevorzugt 0,001 bis 0,38 Gew.-%, weiterhin besonders bevorzugt 0,001 bis 0,34 Gew.-% gewählt wird.

Aluminium ist ein wichtiges Desoxidationsmittel, insbesondere in Stählen mit niedrigen Mangan- und Siliziumgehalten. Durch die Bildung von Aluminiumnitriden kann die austenitische Kornvergröberung beim Warmwalzen kontrolliert werden. Zu hohe Aluminiumgehalten führen allerdings zu einer Vergröberung von aluminatartigen Einschlüssen im Stahl, die insbesondere für die Zähigkeit schädlich sein können.

Es ist vorteilhaft, wenn ein Aluminiumgehalt von 0,0005 bis 0,1 Gew.-%, vorzugsweise 0,0005 bis 0,095 Gew.-%, besonders bevorzugt 0,0005 bis 0,092 Gew.-%, weiterhin besonders bevorzugt 0,0005 bis 0,09 Gew.-% gewählt wird.

Bor verbessert die Härtbarkeit und senkt bei Cu-legierten Stählen die Gefahr der Rotbrüchigkeit. Durch Seigerungen an den früheren austenitischen Korngrenzen erhöht Bor zudem die Widerstandsfähigkeit gegen Wasserstoffbruch. Bor wirkt in Synergie mit Molybdän und Niob und erhöht so die Wirksamkeit dieser Elemente. Höhere Borgehalte sind jedoch zu vermeiden, da diese zur Bildung von spröden Eisenborkarbiden führen.

Es ist vorteilhaft, wenn ein Borgehalt von 0,0005 bis 0,01 Gew.-%, vorzugsweise 0,0005 bis 0,009 Gew.-%, besonders bevorzugt 0,0005 bis 0,008 Gew.-%, weiterhin besonders bevorzugt 0,0005 bis 0,007 Gew.-% gewählt wird.

Titan kann unerwünschten Stickstoff abbinden und trägt zur Ausscheidungshärtung bei. Es erhöht auch die verzögerte Bruchfestigkeit. Nachteilig verbunden mit höheren Ti-Gehalten ist die Abbindung des gelösten Bors in Form von Titanboriden, die sich bereits bei hohen Temperaturen bilden.

Es ist vorteilhaft, wenn ein Titangehalt von 0,001 bis 0,08 Gew.-%, vorzugsweise 0,001 bis 0,075 Gew.-%, besonders bevorzugt 0,001 bis 0,072 Gew.-%, weiterhin besonders bevorzugt 0,001 bis 0,07 Gew.-% gewählt wird.

Niob erhöht die Wasserstoffbeständigkeit und unterstützt eine gute Verteilung schädlicher Elemente wie Phosphor und Schwefel im Stahl. Hohe Niobgehalte führen jedoch zu großen Ausscheidungen, die die Beständigkeit des Stahls gegen Spätbruch beeinträchtigen. Außerdem führt ein zu hoher Niobgehalt zu einem erhöhten Risiko von "Riss"-Fehlern an der Oberfläche der Knüppel und Vorblöcke beim Stranggießen. Diese Defekte können insbesondere die Dauerfestigkeit und die Wasserstoffbeständigkeit negativ beeinflussen.

Es ist vorteilhaft, wenn ein Niobgehalt von 0,001 bis 0,1 Gew.-%, vorzugsweise 0,001 bis 0,095 Gew.-%, besonders bevorzugt 0,001 bis 0,092 Gew.-%, weiterhin besonders bevorzugt 0,001 bis 0,09 Gew.-% gewählt wird.

In geringen Mengen kann Stickstoff dazu beitragen, eine übermäßige Vergröberung des Austenitkorns während der Wärmebehandlung des Stahls zu vermeiden. Zudem ermöglicht die Bildung von Karbonitriden den Einschluss von Wasserstoff. Als Austenitbildner kann Stickstoff Nickel und Kohlenstoff teilweise substituieren. In Bor-legierten Stählen bindet Stickstoff Bor durch die Bildung von Bornitriden, sodass die positive Wirkung von Bor auf die Härtbarkeit des Stahls verloren geht. Daher ist der Stickstoffgehalt im borlegierten Stählen auf 0,01 Gew.-% begrenzt.

Es ist vorteilhaft, wenn ein Stickstoffgehalt von 0,0001 bis 0,05 Gew.-%, vorzugsweise 0,0001 bis 0,045 Gew.-%, besonders bevorzugt 0,0001 bis 0,042 Gew.-%, weiterhin besonders bevorzugt 0,0001 bis 0,04 Gew.-% gewählt wird.

Die Wirkung von Phosphor und Schwefel ist in Vergütungsstählen besonders ungünstig, da diese Elemente die Wasserstoffrekombination behindern und so zu einer höheren Konzentration atomaren Wasserstoffs im Material führen, was das Risiko verzögerten Bruchs erhöht. Zudem bedingen Phosphor und Schwefel Seigerungen an den Korngrenzen (Gefahr von Korngrenzenrissen) und erhöhen das Risiko für Heißrisse. Die Gehalte von Phosphor und Schwefel müssen daher sehr niedrig gehalten werden.

Es ist zu beachten, dass die schädliche Wirkung von Einflusselementen vor allem in Kombination untereinander als kritisch zu sehen ist, wobei deren Ursprung in entsprechenden Einsatzmaterialverunreinigung (beim Einsatzmix zur Erstellung der Schmelze) begründet ist. Bei Verwendung von Schmelztechnologien mit erhöhten Mengen an Sekundärrohstoffen können somit durch die unweigerlich erhöhte Einflusselementfracht größere Schwankungsbreiten bezüglich der werkstoffkundlichen, insbesondere mechanischen Eigenschaft auftreten.

Die Erfindung betrifft somit ein Verfahren zur Herstellung von Vergütungsstählen für Ölfeldanwendungen mit spezifizierten werkstoffkundlichen Eigenschaften, welche durch eine Ziellegierung sichergestellt werden, wobei zumindest ein primärmetallurgisches Aggregat zu einer ersten Schmelze führt, wobei aus der ersten Schmelze zumindest eine Analyse von Gehalten der Elemente durchgeführt wird, wobei zumindest ein Ist-Wert der Schmelzanalyse an ein Steuersystem geleitet wird, wobei zwischen Einfluss- und Kompensationselementen unterschieden wird, wobei Einflusselemente Elemente sind, welche einen Einfluss auf zumindest eine werkstoffkundliche Eigenschaft eines Endprodukts haben, und Kompensationselemente Elemente sind, die einen Einfluss auf die zumindest eine werkstoffkundliche Eigenschaft haben und den Einfluss der Einflusselemente kompensieren, wobei die Größe des Einflusses des zumindest einen Ist-Werts der jeweiligen Einfluss- und Kompensationselemente auf die zumindest eine werkstoffkundliche Eigenschaft des Endprodukts bekannt ist, wobei der zumindest eine Ist-Wert bezüglich derjenigen Einfluss- und Kompensationselemente erfasst wird, welche einen Einfluss auf die zumindest eine werkstoffkundliche Eigenschaft des Endprodukts haben, wobei zur zumindest einen werkstoffkundlichen Eigenschaft des Endprodukts zumindest ein Kompensationselement ermittelt wird, welches die zumindest eine werkstoffkundliche Eigenschaft einstellt, wobei eines oder mehrere Kompensationselemente in die Schmelze in einer Menge zulegiert werden, welche die zumindest eine werkstoffkundliche Eigenschaft in einen Zielbereich bringt und den Einfluss eines oder mehrerer Einflusselemente auf die zumindest eine werkstoffkundliche Eigenschaft kompensiert, und wobei hierfür im Steuersystem zwei Komponenten verwendet werden: ein Prognosemodell und ein Korrekturmodell, wobei mit dem Prognosemodell der Einfluss der Einfluss- und Kompensationselemente auf die zumindest eine werkstoffkundliche Eigenschaft des Endprodukts und mit dem Korrekturmodell korrigierte Zielwerte der Kompensationselemente berechnet werden, um die zumindest eine werkstoffkundliche Eigenschaft in einen Zielbereich zu bringen.

Die Menge der zulegierten Kompensationselemente kann im Sinne der Erfindung auch 0 betragen, wenn dies für die Kompensation der Wirkung bzw. des Einflusses der Einflusselemente für nötig erachtet wird.

Eine Weiterbildung sieht vor, dass die werkstoffkundlichen Eigenschaften Hollomon-Jaffe-Parameter, Härtbarkeitsindizes, ein oder mehrere Kohlenstoffäquivalente, Dehngrenze YS und Zugfestigkeit TS umfassen.

Eine Weiterbildung sieht vor, dass die Analyse mittels Probenentnahme nach EN ISO 14284 durchgeführt wird.

Eine Weiterbildung sieht vor, dass auf die Stahlherstellung folgende Nachbehandlungsschritte bis zum Erhalt des Endprodukts unverändert durchgeführt werden.

Eine Weiterbildung sieht vor, dass das Prognosemodell und/oder Korrekturmodell unter Verwendung eines, mehrerer oder aller nachfolgender Modelle erzeugt wird: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

Eine Weiterbildung sieht vor, dass das Prognosemodell und/oder Korrekturmodell unter Verwendung von linearen Regressionsmodellen, polynomialen Regressionsmodellen, Decision-Tree-Based Regressionsmodellen, Random-Forest-Regressionsmodellen, Nearest-Neighbour-Modellen, Neuronal-Network-Modellen, Support-Vector-Machine-Modellen, ADA-Boost-Modellen, Regression-Boost-Modellen, HIST-Boost-Modellen, XGBOOST-Modellen, Generalized-Additive-Modellen und/oder Symbolic-Regression-Modelle erzeugt wird.

Eine Weiterbildung sieht vor, dass, wenn eine werkstoffkundliche Eigenschaft betrachtet wird, der zumindest eine erfasste Ist-Wert der Analyse unter Heranziehung der Quadratwurzel der mittleren quadratischen Abweichung der werkstoffkundlichen Eigenschaft dem zumindest einen Prognosemodell und/oder Korrekturmodell zugeordnet wird, wobei für den zumindest einen Ist-Wert die Quadratwurzel der mittleren quadratischen Abweichung der werkstoffkundlichen Eigenschaft für alle Prognosemodelle und/oder Korrekturmodelle ermittelt wird und basierend auf den Ergebnissen jenes Prognosemodell und/oder Korrekturmodell mit der höchsten Genauigkeit gewählt wird.

Eine Weiterbildung sieht vor, dass, wenn mehrere werkstoffkundliche Eigenschaften betrachtet werden, der zumindest eine erfasste Ist-Wert der Analyse unter Heranziehung des Mittelwerts und/oder der Summe der Quadratwurzeln der mittleren quadratischen Abweichungen der werkstoffkundlichen Eigenschaften dem zumindest einen Prognosemodell und/oder Korrekturmodell zugeordnet wird, wobei für den zumindest einen Ist-Wert der Mittelwert und/oder die Summe der Quadratwurzeln der mittleren quadratischen Abweichungen der werkstoffkundlichen Eigenschaften für alle Prognosemodelle und/oder Korrekturmodelle ermittelt wird und basierend auf den Ergebnissen jenes Prognosemodell und/oder Korrekturmodell mit der höchsten Genauigkeit gewählt wird.

Eine Weiterbildung sieht vor, dass das Prognosemodell und/oder Korrekturmodell unter Verwendung einer multiplen linearen Regressionsanalyse erzeugt wird.

Eine Weiterbildung sieht vor, dass die Zugfestigkeit TS und die Dehngrenze YS in einem auf multipler linearer Regressionsanalyse basierenden Prognose- und/oder Korrekturmodell gemäß den folgenden Formeln berechnet werden: $TS = {α}_{0} + {α}_{1} \times {X}_{1} + α \times {X}_{2} + ⋯ + {α}_{n} \times {X}_{n} ;$ $YS = {β}_{0} + {β}_{1} \times {X}_{1} + {β}_{2} \times {X}_{2} + ⋯ + {β}_{n} \times {X}_{n} ,$ wobei
- α₀ und β₀: Basisfaktoren der Zugfestigkeit TS und Dehngrenze YS,
- α₁- αₙ und β₁- βₙ: Regressionskoeffizienten und
- X₁-Xₙ: Elementgehalte in Gew.-% sind.

Eine Weiterbildung sieht vor, dass die Einflusselemente, deren Einfluss auf werkstoffkundliche Eigenschaften des Endprodukts kompensiert werden soll, eines, mehrere oder alle aus der Gruppe von Ni, Mo, Cu umfassen.

Eine Weiterbildung sieht vor, dass die Einflusselemente, deren Einfluss auf werkstoffkundliche Eigenschaften des Endprodukts kompensiert werden soll, Ni, Mo und/oder Cu umfassen.

Eine Weiterbildung sieht vor, dass bei Ni-legierten Vergütungsstählen Mo und Cu, bei Mo-legierten Vergütungsstählen Ni und Cu und bei Ni-Mo-legierten Vergütungsstählen Cu Einflusselemente sind, wobei in diesen Fällen entsprechend die zuvor genannten Legierungselemente auch als Kompensationselemente geeignet sind.

Eine Weiterbildung sieht vor, dass als Kompensationselemente eines, mehrere oder alle aus der Gruppe von C, Si, Mn, Cr ausgewählt werden.

Eine Weiterbildung sieht vor, dass als Kompensationselemente C, Si, Mn und/oder Cr ausgewählt werden.

Eine Weiterbildung sieht vor, dass zur Berechnung des Kohlenstoffäquivalents in Abhängigkeit von Normen und/oder Spezifikationen einer bestimmten Vergütungsstahlgüte zumindest eine der folgenden Formeln verwendet wird: $\begin{matrix}CEV \left(Carbon Equivalent Value\right) = Gew . - % C + \frac{Gew . - % Mn}{6} + \frac{Gew . - % Cr + Gew . - % Mo + Gew . - % V}{5} + \\ \frac{Gew . - % Ni + Gew . - % Cu}{15} ;\end{matrix}$ $\begin{matrix}CET \left(Carbon Equivalent Thyssen\right) = Gew . - % C + \frac{Gew . - % Mn + Gew . - % Mo}{10} + \frac{Gew . - % Cr + Gew . - % Cu}{20} + \\ \frac{Gew . - % Ni}{40} ;\end{matrix}$ $\begin{matrix}Pcm \left(Critical Metal Parameter\right) = Gew . - % C + \frac{Gew . - % Si}{30} + \frac{Gew . - % Mn + Gew . - % Cu + Gew . - % Cr}{20} + \\ \frac{Gew . - % Mo}{15} + \frac{Gew . - % Ni}{60} + \frac{Gew . - % V}{10} + 5 \times Gew . - %B ,\end{matrix}$ wobei Gew.-%i der Gehalt des jeweiligen Elements i in Gew.-% ist.

Eine Weiterbildung sieht vor, dass die Analyse zumindest in der ersten Schmelze des letzten primärmetallurgischen Aggregates einer Erzeugungsroute, insbesondere ab einem Abstich von Rohstahl, vor oder nach einer ersten Zugabe von Legierungselementen und zumindest nach einer zweiten Zugabe von Legierungselementen durchgeführt wird.

Eine Weiterbildung sieht vor, dass, wenn ein erstes Element durch einen Einsatzstoff in einem Anteil eingebracht wird, der unterhalb eines gewünschten, durch die Ziellegierung definierten Zielbereichs liegt, das erste Element, sofern es ein gewünschtes Legierungselement ist, in dem Umfang zulegiert wird, der zum Erreichen des Zielbereichs notwendig ist, oder, wenn das erste Element durch einen Einsatzstoff in einem Anteil eingebracht wird, der bereits im Zielbereich entspricht, das erste Element nicht mehr zulegiert wird oder, wenn das erste Element durch einen Einsatzstoff in einem Anteil eingebracht wird, der über dem Zielbereich liegt, das erste Element durch einen verringerten Legierungsanteil eines zweiten ähnlich wirkenden Elements oder einen erhöhten Legierungsanteil eines gegenläufig wirkenden dritten Elements kompensiert wird.

Eine Weiterbildung sieht vor, dass das verwendete Korrekturmodell eine Reihung der unterschiedlichen Kompensationselemente nach auflösungs- und ausbringungsspezifischen sowie ökonomischen Gesichtspunkten festlegt.

Der Begriff "Auflösung" bezieht sich auf die Auflösung der Einsatzstoffe in der Schmelze. Dies bedeutet in diesem Zusammenhang, dass im Zweifel ein Element als Kompensationselement eher nicht berücksichtigt wird, wenn sein Auflösen in der Schmelze zu lange dauert und insbesondere den gesamten Prozess verzögern würde.

Der Begriff "Ausbringung" bezieht sich auf die Ausbeute.

Eine Weiterbildung sieht vor, dass die Ziellegierung die folgende Ziellegierungszusammensetzung in Gew.-% umfasst:

| | |
|---|---|
| C | 0,05-1,2 |
| Si | 0,001-1,9 |
| Mn | 0,1-3,5 |

zudem Eisen und unvermeidbare Verunreinigungen,
wobei lediglich optional eines, mehrere oder alle der folgenden Elemente in Gew.-% enthalten sind:

| | |
|---|---|
| P | ≤ 0,1 |
| S | ≤0,35 |
| Cr | 0,001-15 |
| Cu | 0,001-0,5 |
| Mo | 0,001-1,5 |
| Ni | 0,001-4,0 |
| N | 0,0001-0,05 |
| Sn | 0,001-0,03 |
| V | 0,001-0,4 |
| Nb | 0,001-0,1 |
| Ti | 0,001-0,08 |
| Al | 0,0005-0,1 |
| B | 0,0005-0,01. |

Eine Weiterbildung sieht vor, dass die Ziellegierung die folgende Ziellegierungszusammensetzung in Gew.-% umfasst:

| | |
|---|---|
| C | 0,05-1,2 |
| Si | 0,001-1,9 |
| Mn | 0,1-3,5 |

zudem Eisen und unvermeidbare Verunreinigungen,
wobei lediglich optional folgende Elemente in Gew.-% enthalten sind:

| | |
|---|---|
| P | ≤ 0,1 |
| S | ≤0,35 |
| Cr | 0,001-15 |
| Cu | 0,001-0,5 |
| Mo | 0,001-1,5 |
| Ni | 0,001-4,0 |
| N | 0,0001-0,05 |
| Sn | 0,001-0,03 |
| V | 0,001-0,4 |
| Nb | 0,001-0,1 |
| Ti | 0,001-0,08 |
| Al | 0,0005-0,1 und/oder |
| B | 0,0005-0,01. |

Die Erfindung betrifft ferner ein Steuersystem zur Herstellung von Vergütungsstählen für Ölfeldanwendungen mit spezifizierten werkstoffkundlichen Eigenschaften, welche durch eine Ziellegierung sichergestellt werden, und zur Durchführung des erfindungsgemäßen Verfahrens, wobei das Steuersystem zwei Komponenten umfasst: ein Prognosemodell und ein Korrekturmodell, wobei das Prognosemodell dazu ausgelegt ist, eine Wirkung von Einflusselementen und Kompensationselementen auf zumindest eine werkstoffkundliche Eigenschaften des Vergütungsstahls zu berechnen, und das Korrekturmodell dazu ausgelegt ist, korrigierte Zielwerte von Kompensationselementen zu berechnen, um die zumindest eine werkstoffkundliche Eigenschaft in einen Zielbereich zu bringen.

Eine Weiterbildung sieht vor, dass das Prognosemodell und/oder Korrekturmodell unter Verwendung eines, mehrerer oder aller nachfolgender Modelle erzeugbar ist: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

Eine Weiterbildung sieht vor, dass das Prognosemodell und/oder Korrekturmodell unter Verwendung von linearen Regressionsmodellen, polynomialen Regressionsmodellen, Decision-Tree-Based Regressionsmodellen, Random-Forest-Regressionsmodellen, Nearest-Neighbour-Modellen, Neuronal-Network-Modellen, Support-Vector-Machine-Modellen, ADA-Boost-Modellen, Regression-Boost-Modellen, HIST-Boost-Modellen, XGBOOST-Modellen, Generalized-Additive-Modellen und/oder Symbolic-Regression-Modelle erzeugbar ist.

Eine Weiterbildung sieht vor, dass die werkstoffkundlichen Eigenschaften Zugfestigkeit TS und Dehngrenze YS in einem auf multipler linearer Regressionsanalyse basierenden Prognose- und/oder Korrekturmodell gemäß den folgenden Formeln berechenbar sind: $TS = {α}_{0} + {α}_{1} \times {X}_{1} + α \times {X}_{2} + ⋯ + {α}_{n} \times {X}_{n} ;$ $YS = {β}_{0} + {β}_{1} \times {X}_{1} + {β}_{2} \times {X}_{2} + ⋯ + {β}_{n} \times {X}_{n} ,$ wobei
- α₀ und β₀: Basisfaktoren der Zugfestigkeit TS und Dehngrenze YS,
- α₁- αₙ und β₁- βₙ: Regressionskoeffizienten und
- X₁-Xₙ: Elementgehalte in Gew.-% sind.

Eine Weiterbildung sieht vor, dass zur Berechnung eines Kohlenstoffäquivalent in Abhängigkeit von Normen und/oder Spezifikationen einer bestimmten Vergütungsstahlgüte zumindest eine der folgenden Formeln verwendbar ist: $\begin{matrix}CEV \left(Carbon Equivalent Value\right) = Gew . - % C + \frac{Gew . - % Mn}{6} + \frac{Gew . - % Cr + Gew . - % Mo + Gew . - % V}{5} + \\ \frac{Gew . - % Ni + Gew . - % Cu}{15} ;\end{matrix}$ $\begin{matrix}CET \left(Carbon Equivalent Thyssen\right) = Gew . - % C + \frac{Gew . - % Mn + Gew . - % Mo}{10} + \frac{Gew . - % Cr + Gew . - % Cu}{20} + \\ \frac{Gew . - % Ni}{40} ;\end{matrix}$ $\begin{matrix}Pcm \left(Critical Metal Parameter\right) = Gew . - % C + \frac{Gew . - % Si}{30} + \frac{Gew . - % Mn + Gew . - % Cu + Gew . - % Cr}{20} + \\ \frac{Gew . - % Mo}{15} + \frac{Gew . - % Ni}{60} + \frac{Gew . - % V}{10} + 5 \times Gew . - %B ,\end{matrix}$ wobei Gew.-%i der Gehalt des jeweiligen Elements i in Gew.-% ist.

Eine Weiterbildung sieht vor, dass die Ziellegierung die folgende Ziellegierungszusammensetzung in Gew.-% umfasst:

| | |
|---|---|
| C | 0,05-1,2 |
| Si | 0,001-1,9 |
| Mn | 0,1-3,5 |

zudem Eisen und unvermeidbare Verunreinigungen,
wobei lediglich optional eines, mehrere oder alle der folgenden Elemente in Gew.-% enthalten sind:

| | |
|---|---|
| P | ≤ 0,1 |
| S | ≤0,35 |
| Cr | 0,001-15 |
| Cu | 0,001-0,5 |
| Mo | 0,001-1,5 |
| Ni | 0,001-4,0 |
| N | 0,0001-0,05 |
| Sn | 0,001-0,03 |
| V | 0,001-0,4 |
| Nb | 0,001-0,1 |
| Ti | 0,001-0,08 |
| Al | 0,0005-0,1 |
| B | 0,0005-0,01. |

Eine Weiterbildung sieht vor, dass die Ziellegierung die folgende Ziellegierungszusammensetzung in Gew.-% umfasst:

| | |
|---|---|
| C | 0,05-1,2 |
| Si | 0,001-1,9 |
| Mn | 0,1-3,5 |

zudem Eisen und unvermeidbare Verunreinigungen,
wobei lediglich optional folgende Elemente in Gew.-% enthalten sind:

| | |
|---|---|
| P | ≤ 0,1 |
| S | ≤0,35 |
| Cr | 0,001-15 |
| Cu | 0,001-0,5 |
| Mo | 0,001-1,5 |
| Ni | 0,001-4,0 |
| N | 0,0001-0,05 |
| Sn | 0,001-0,03 |
| V | 0,001-0,4 |
| Nb | 0,001-0,1 |
| Ti | 0,001-0,08 |
| Al | 0,0005-0,1 und/oder |
| B | 0,0005-0,01. |

Die Erfindung betrifft ferner einen metallischen Hohlkörper, insbesondere ein nahtloses Stahlrohr für Ölfeldanwendungen aus einem Vergütungsstahl, hergestellt nach dem erfindungsgemäßen Verfahren.

Die Erfindung wird beispielhaft anhand einer Zeichnung dargestellt. Es zeigen dabei:
- Figur 1: den Prozessablauf des dynamischen Legierens;
- Figur 2: beispielhafte Untergrenzen bzw. Minimalgehalte (Min), Standardabweichungen (SD), Durchschnittswerte (Avg.) und Obergrenzen bzw. Maximalgehalte (Max) für Dehngrenze, Zugfestigkeit, Einfluss- und Kompensationselemente;
- Figur 3: beispielhafte Zugfestigkeits- und Dehngrenzen-Werte sowie Elementgehalte im Rahmen des erfindungsgemäßen dynamischen Legierens, zeigend die Kompensation der Wirkungen der Einflusselemente durch Adaption der Kompensationselementgehalte;
- Figur 4: PCA (Hauptkomponentenanalyse, Principal Component Analysis) zur Abschätzung des Einflusses der einzelnen Elemente für das Beispiel L80-1 (API 5-CT);
- Figur 5: Beispiel L80-1 - Anwendung der Regression auf Trainingsdaten: prognostizierte YS-Werte (YS - prog.) vs. beobachtete bzw. gemessene YS-Werte (YS - beob.);
- Figur 6: Beispiel L80-1 - Anwendung der Regression auf die Grundgesamtheit: prognostizierte YS-Werte (YS - prog.) vs. beobachtete bzw. gemessene YS-Werte (YS - beob.);
- Figur 7: Beispiel L80-1 - Anwendung der Regression auf Trainingsdaten: prognostizierte TS-Werte (TS - prog.) vs. beobachtete bzw. gemessene TS-Werte (TS - beob.);
- Figur 8: Beispiel L80-1 - Anwendung der Regression auf die Grundgesamtheit: prognostizierte TS-Werte (TS - prog.) vs. beobachtete bzw. gemessene TS-Werte (TS - beob.);
- Figur 9: Beispiel L80-1 - Vergleich zwischen Prognose und Beobachtung bzw. Messung;
- Figur 10: PCA (Hauptkomponentenanalyse, Principal Component Analysis) zur Abschätzung des Einflusses der einzelnen Elemente für das Beispiel P110 (API 5-CT);
- Figur 11: Beispiel P110 - Anwendung der Regression auf Trainingsdaten: prognostizierte YS-Werte (YS - prog.) vs. beobachtete bzw. gemessene YS-Werte (YS - beob.);
- Figur 12: Beispiel P110 - Anwendung der Regression auf die Grundgesamtheit: prognostizierte YS-Werte (YS - prog.) vs. beobachtete bzw. gemessene YS-Werte (YS - beob.);
- Figur 13: Beispiel P110 - Anwendung der Regression auf Trainingsdaten: prognostizierte TS-Werte (TS - prog.) vs. beobachtete bzw. gemessene TS-Werte (TS - beob.);
- Figur 14: Beispiel P110 - Anwendung der Regression auf die Grundgesamtheit: prognostizierte TS-Werte (TS - prog.) vs. beobachtete bzw. gemessene TS-Werte (TS - beob.);
- Figur 15: Beispiel P110 - Vergleich zwischen Prognose und Beobachtung bzw. Messung.

Die Erfinder haben erkannt, dass es besonders vorteilhaft ist, zwischen Einfluss- und Kompensationselementen zu unterscheiden.

Die Wirkung bzw. der Einfluss der Einflusselemente wird durch das Zulegieren von Kompensationselementen kompensiert.

Die Wirkung bzw. der Einfluss der in der Schmelze bereits vorhandenen Einflusselemente auf die werkstoffkundliche, insbesondere mechanische Eigenschaft des Vergütungsstahlprodukts wird mit einem Prognosemodell modelliert. Eine gegebenenfalls notwendige Korrektur wird mit einem Korrekturmodell ermittelt.

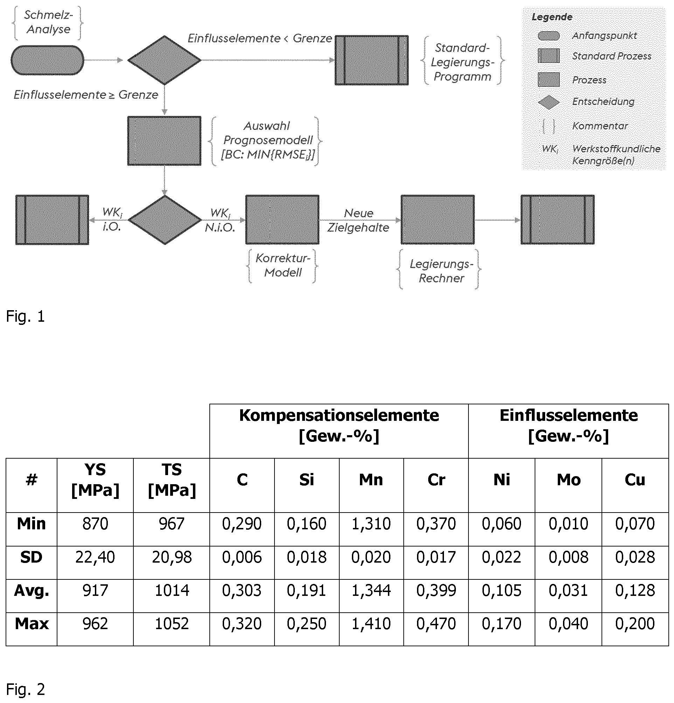

Figur 1 illustriert in vereinfachter Art und Weise das erfindungsgemäße Verfahren. Ausgehend von einer Schmelzanalyse wird entschieden, ob die Analyse innerhalb der spezifizierten Grenzen für die Einflusselementgehalte liegt oder sich davon unterscheidet. Wenn die Einflusselementgehalte innerhalb der Grenzen liegen, wird der Standard-Prozess durchgeführt, d.h. es kommt nicht zum dynamischen Legieren. Liegen die Einflusselementgehalte jedoch außerhalb der spezifizierten Grenzen, wird mittels des Prognosemodells der Einfluss auf zumindest eine spezifizierte werkstoffkundliche, insbesondere mechanische Eigenschaft ermittelt, um festzustellen, ob die prognostizierte Eigenschaft innerhalb oder außerhalb des durch die gewünschte Vergütungsstahlgüte definierten Zielbereichs liegt. Liegt die Eigenschaft im spezifizierten Zielbereich, wird der Standard-Prozess durchgeführt, d.h. es kommt nicht zum dynamischen Legieren. Wenn die prognostizierte Eigenschaft jedoch außerhalb des spezifizierten Zielbereichs liegt, werden mithilfe des Korrekturmodells adaptierte Legierungselementgehalte berechnet, sodass durch entsprechende Zugabe von Kompensationselementen die Wirkung der Einflusselemente dahingehend kompensiert werden kann, dass die betreffende Eigenschaft in den spezifizierten Zielbereich gebracht wird. Anschließend wird wieder nach dem Standard-Prozess verfahren. Nach dem dynamischen Legieren können gegebenenfalls auch erneut Analyse-Ist-Werte in das Prognosemodell eingespeist werden. Liegt die prognostizierte Eigenschaft im spezifizierten Zielbereich, wird nachfolgend nach dem Standard-Prozess verfahren. Liegt die prognostizierte Eigenschaft jedoch immer noch nicht im spezifizierten Zielbereich, kann erneut dynamisches Legieren durchgeführt werden.

In Figur 2 sind beispielhafte Untergrenzen bzw. Minimalgehalte (Min), Standardabweichungen (SD), Durchschnittswerte (Avg.) und Obergrenzen bzw. Maximalgehalte (Max) für Dehngrenze, Zugfestigkeit, Einfluss- und Kompensationselemente gezeigt.

Zu den Einflusselementen zählen hier Ni, Mo und Cu. Die Kompensationselemente sind C, Si, Mn und Cr.

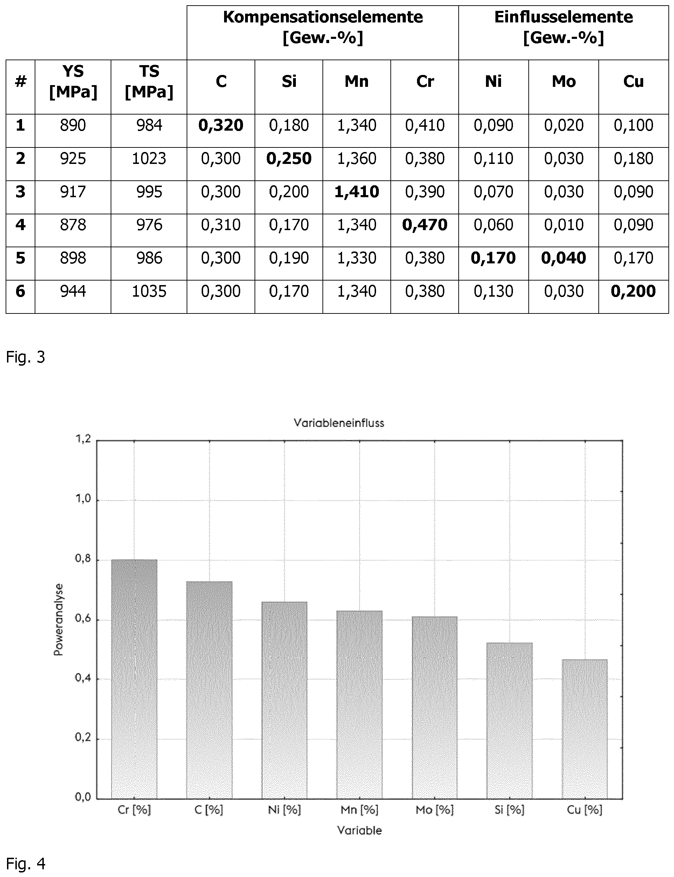

Figur 3 zeigt für sechs beispielhafte Fälle des erfindungsgemäßen dynamischen Legierens Zugfestigkeits- und Dehngrenzen-Werte sowie Elementgehalte. Um den Einfluss der variablen Einflusselementgehalte zu kompensieren, werden durch das Korrekturmodell adaptierte Kompensationselementgehalte berechnet. Im Hinblick auf die beobachteten bzw. gemessenen TS- und YS-Werte wird ersichtlich, dass sich diese aufgrund der Kompensation der Wirkung der Einflusselemente durch Adaption der Kompensationselementgehalte trotz variablen Einflusselementgehalten im jeweiligen Zielbereich halten lassen.

Nachfolgend wird die hinreichende Genauigkeit der im Rahmen des erfindungsgemäßen Verfahrens verwendeten Modelle noch anhand von zwei konkreten Beispielen, zwei unterschiedlichen Vergütungsstahllegierungen, veranschaulicht.

### Beispiel 1: L80-1 (API 5-CT)

Figur 4 zeigt das Ergebnis der PCA (Hauptkomponentenanalyse, Principal Component Analysis). Hierdurch wird verifiziert ob und wie maßgeblich eine Variable einen Einfluss auf das Ergebnis hat.

Durch multiple lineare Regressionsanalyse werden Formeln für die Dehngrenze YS und Zugfestigkeit TS bestimmt: $\begin{matrix}YS = 644 - 102 \times Gew . - %C - 174 \times Gew . - %Mn + 540 \times Gew . - %Cr + 330 \times Gew . - %Ni + \\ 915 \times Gew . - %Mo - 495 \times Gew . - %Cu + 146 \times Gew . - %Si ;\end{matrix}$ $\begin{matrix}TS = 543 + 955 \times Gew . - %C - 123 \times Gew . - %Mn + 112 \times Gew . - %Cr + 185 \times Gew . - %Ni + \\ 1448 \times Gew . - %Mo + 324 \times Gew . - %Cu + 158 \times Gew . - %Si .\end{matrix}$

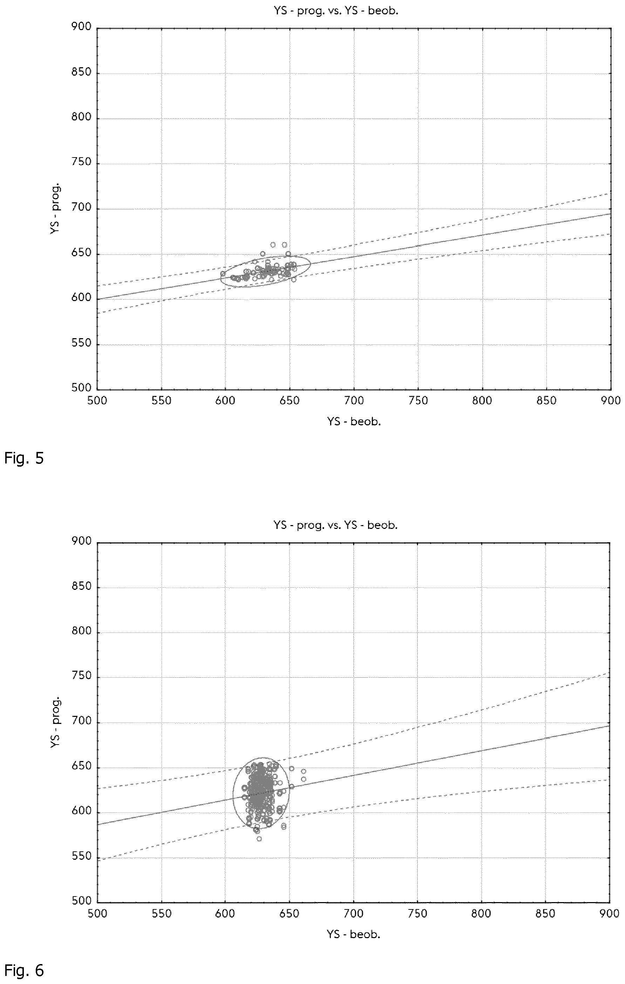

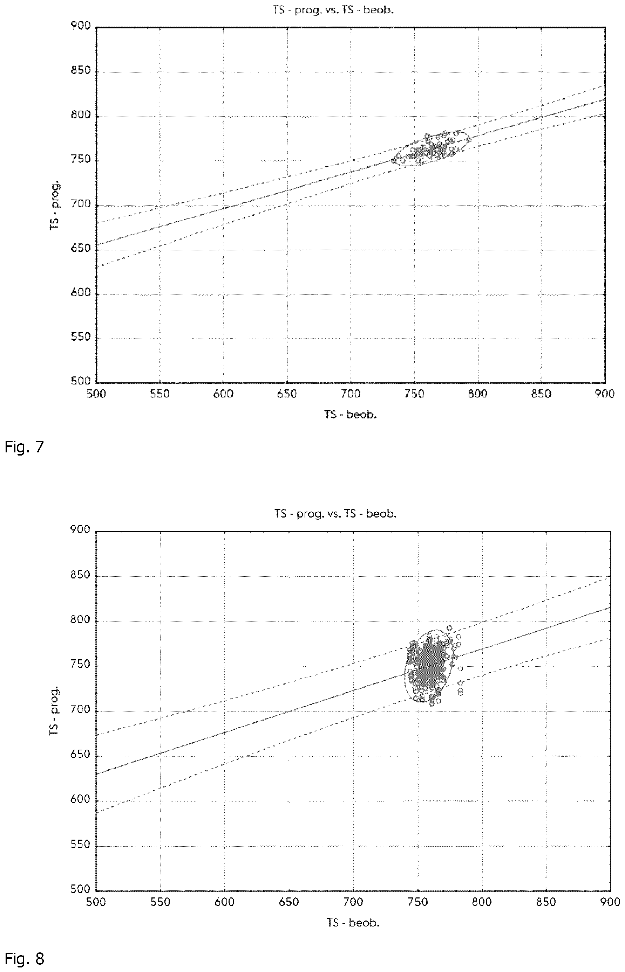

Die Figuren 5 bis 8 zeigen Auftragungen der prognostizierten Werte (prog.) gegen die beobachteten bzw. gemessenen Werte (beob.) von YS und TS, wobei die Figuren 5 und 7 auf die Trainingsdaten und die Figuren 6 und 8 auf die Grundgesamtheit bezogen sind. Die Ausgleichsgerade im Kontext zur multiplen linearen Regression ist eine Linie, die die Beziehung zwischen mehreren unabhängigen Variablen und einer abhängigen Variablen beschreibt. Diese Linie minimiert die Summe der quadrierten Abstände (Residuen) zwischen den tatsächlichen Datenpunkten und den vorhergesagten Werten. Die multiple lineare Regression erweitert das Konzept der einfachen linearen Regression, indem sie mehrere Prädiktoren berücksichtigt, um genauere Vorhersagen zu ermöglichen. Die Kreise stellen die prognostizierten Werte dar. Je näher ein Kreis an der Ausgleichsgerade ist, desto besser ist die Prognose. Die 95% Konfidenzbänder (strichlierte Linien) einer Prognosewahrscheinlichkeit geben den Bereich an, in dem erwartet wird, dass 95% der zukünftigen Datenpunkte liegen werden. Diese Bänder berücksichtigen die Unsicherheit in den Schätzungen der Regressionsparameter und bieten eine visuelle Darstellung der Genauigkeit der Vorhersagen. Sie sind breiter als einfache Konfidenzintervalle, da sie die Unsicherheit über den gesamten Bereich der unabhängigen Variablen hinweg berücksichtigen.

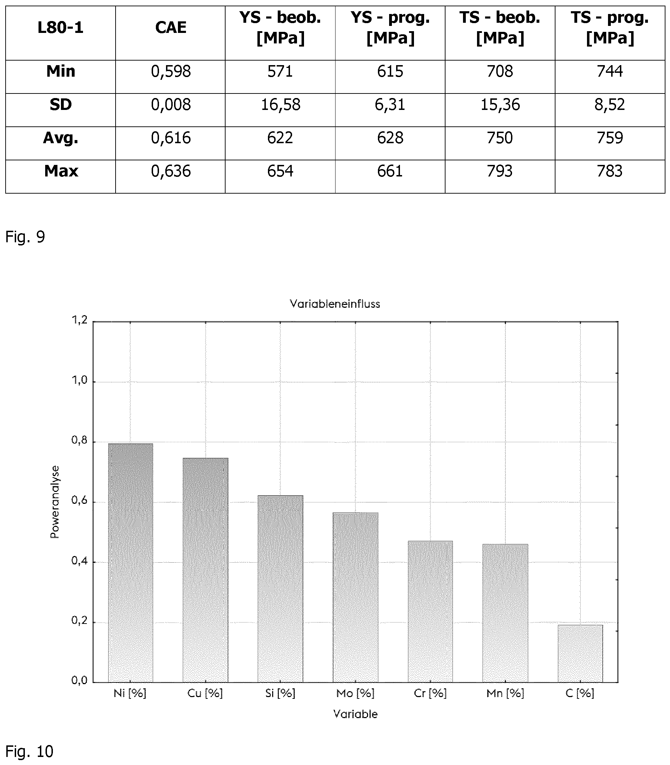

Insgesamt zeigt sich angesichts der guten Vergleichbarkeit der beobachteten bzw. gemessenen und prognostizierten YS- und TS-Werte (siehe Figur 9), dass die im Rahmen des dynamischen Legierens verwendeten Prognose- und/oder Korrekturmodelle präzise Vorhersagen ermöglichen.

### Beispiel 2: P110 (API 5-CT)

Figur 10 zeigt das Ergebnis der PCA (Hauptkomponentenanalyse, Principal Component Analysis). Hierdurch wird verifiziert ob und wie maßgeblich eine Variable einen Einfluss auf das Ergebnis hat.

Durch multiple lineare Regressionsanalyse werden Formeln für die Dehngrenze YS und Zugfestigkeit TS bestimmt: $\begin{matrix}YS = 1126 - 272 \times Gew . - %C - 97 \times Gew . - %Mn + 70 \times Gew . - %Cr + 114 \times Gew . - %Ni + \\ 257 \times Gew . - %Mo - 211 \times Gew . - %Cu + 54 \times Gew . - %Si ;\end{matrix}$ $\begin{matrix}TS = 1240 + 786 \times Gew . - %C - 53 \times Gew . - %Mn + 103 \times Gew . - %Cr + 19 \times Gew . - %Ni + \\ 38 \times Gew . - %Mo + 180 \times Gew . - %Cu + 102 \times Gew . - %Si .\end{matrix}$

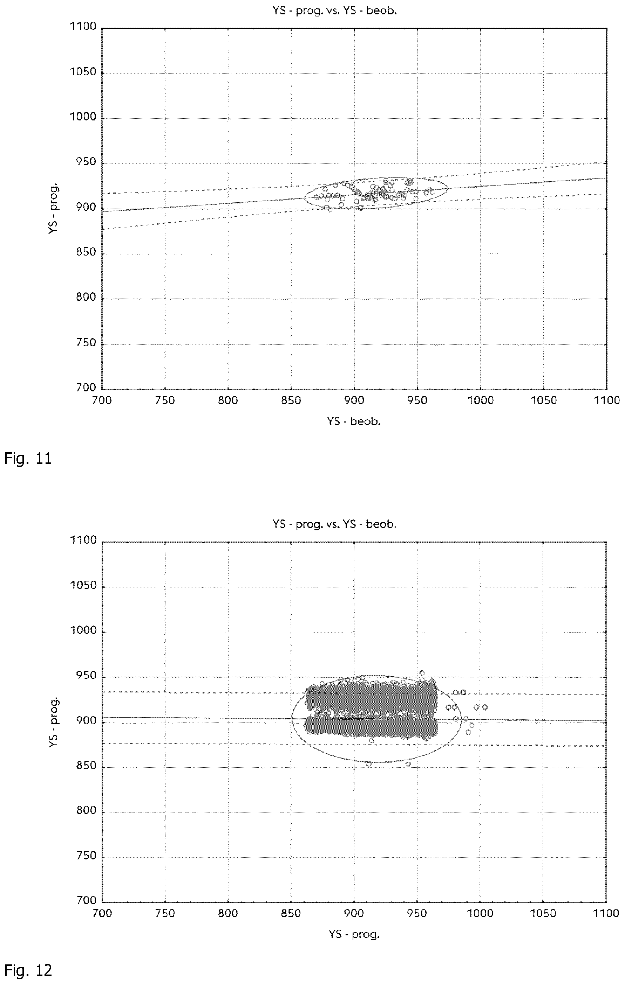

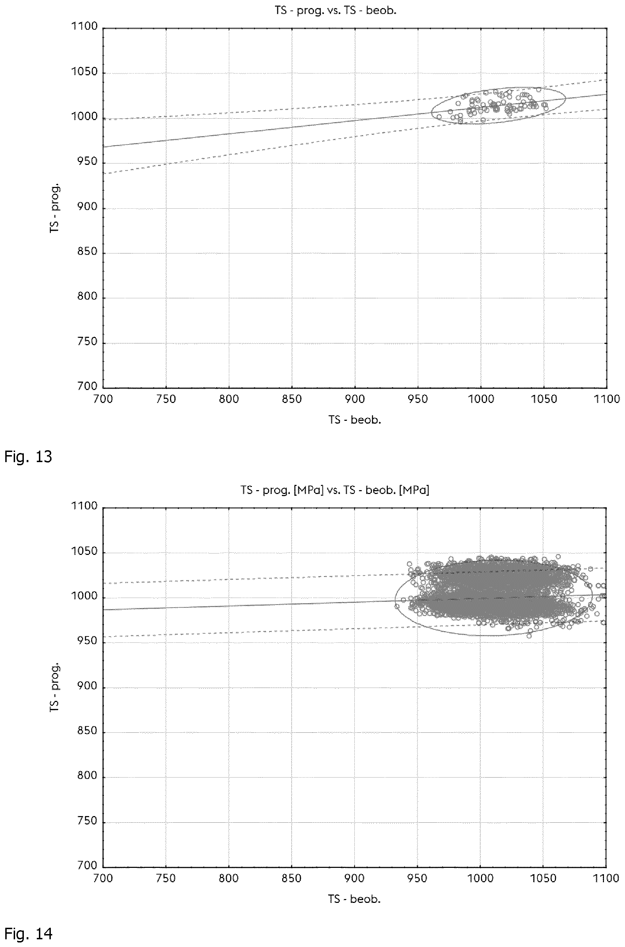

Die Figuren 11 bis 14 zeigen Auftragungen der prognostizierten Werte (prog.) gegen die beobachteten bzw. gemessenen Werte (beob.) von YS und TS, wobei die Figuren 11 und 13 auf die Trainingsdaten und die Figuren 12 und 14 auf die Grundgesamtheit bezogen sind. Die Ausgleichsgerade im Kontext zur multiplen linearen Regression ist eine Linie, die die Beziehung zwischen mehreren unabhängigen Variablen und einer abhängigen Variablen beschreibt. Diese Linie minimiert die Summe der quadrierten Abstände (Residuen) zwischen den tatsächlichen Datenpunkten und den vorhergesagten Werten. Die multiple lineare Regression erweitert das Konzept der einfachen linearen Regression, indem sie mehrere Prädiktoren berücksichtigt, um genauere Vorhersagen zu ermöglichen. Die Kreise stellen die prognostizierten Werte dar. Je näher ein Kreis an der Ausgleichsgerade ist, desto besser ist die Prognose. Die 95% Konfidenzbänder (strichlierte Linien) einer Prognosewahrscheinlichkeit geben den Bereich an, in dem erwartet wird, dass 95% der zukünftigen Datenpunkte liegen werden. Diese Bänder berücksichtigen die Unsicherheit in den Schätzungen der Regressionsparameter und bieten eine visuelle Darstellung der Genauigkeit der Vorhersagen. Sie sind breiter als einfache Konfidenzintervalle, da sie die Unsicherheit über den gesamten Bereich der unabhängigen Variablen hinweg berücksichtigen.

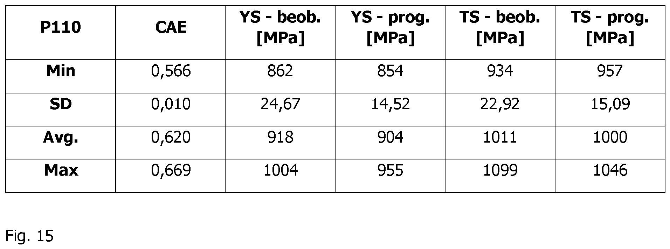

Insgesamt zeigt sich angesichts der guten Vergleichbarkeit der beobachteten bzw. gemessenen und prognostizierten YS- und TS-Werte (siehe Figur 15), dass die im Rahmen des dynamischen Legierens verwendeten Prognose- und/oder Korrekturmodelle präzise Vorhersagen ermöglichen.

Mit dem vorliegenden Verfahren gelingt somit ein dynamisches und flexibles Legieren, bei welchem der Einfluss bzw. die Wirkung der Einflusselemente durch eine gezielte Zugabe der Kompensationselemente kompensiert wird.

Die Kompensationselemente können u.a. unter Berücksichtigung auflösungs- und ausbringungsspezifischer sowie ökonomischer Aspekte ausgewählt werden, sodass hierdurch die Kosteneffizienz des Gesamtverfahrens deutlich erhöht wird.

Außerdem gelingt es mit dem vorliegenden Verfahren, die gewünschten werkstoffkundlichen Eigenschaften trotzt Verwendung von Einsatzstoffen mit erhöhter Einflusselementfracht sicher einzuhalten.

## Patentansprüche

1. Verfahren zur Herstellung von Vergütungsstählen für Ölfeldanwendungen mit spezifizierten werkstoffkundlichen Eigenschaften, welche durch eine Ziellegierung sichergestellt werden, wobei zumindest ein primärmetallurgisches Aggregat zu einer ersten Schmelze führt, wobei aus der ersten Schmelze zumindest eine Analyse von Gehalten der Elemente durchgeführt wird, wobei zumindest ein Ist-Wert der Schmelzanalyse an ein Steuersystem geleitet wird,
**dadurch gekennzeichnet, dass**
zwischen Einfluss- und Kompensationselementen unterschieden wird, wobei
Einflusselemente Elemente sind, welche einen Einfluss auf zumindest eine werkstoffkundliche Eigenschaft eines Endprodukts haben, und Kompensationselemente Elemente sind, die einen Einfluss auf die zumindest eine werkstoffkundliche Eigenschaft haben und den Einfluss der Einflusselemente kompensieren, wobei
die Größe des Einflusses des zumindest einen Ist-Werts der jeweiligen Einfluss- und Kompensationselemente auf die zumindest eine werkstoffkundliche Eigenschaft des Endprodukts bekannt ist, wobei
der zumindest eine Ist-Wert bezüglich derjenigen Einfluss- und Kompensationselemente erfasst wird, welche einen Einfluss auf die zumindest eine werkstoffkundliche Eigenschaft des Endprodukts haben, wobei
zur zumindest einen werkstoffkundlichen Eigenschaft des Endprodukts zumindest ein Kompensationselement ermittelt wird, welches die zumindest eine werkstoffkundliche Eigenschaft einstellt, wobei
eines oder mehrere Kompensationselemente in die Schmelze in einer Menge zulegiert werden, welche die zumindest eine werkstoffkundliche Eigenschaft in einen Zielbereich bringt und den Einfluss eines oder mehrerer Einflusselemente auf die zumindest eine werkstoffkundliche Eigenschaft kompensiert, und wobei
hierfür im Steuersystem zwei Komponenten verwendet werden: ein Prognosemodell und ein Korrekturmodell, wobei mit dem Prognosemodell der Einfluss der Einfluss- und Kompensationselemente auf die zumindest eine werkstoffkundliche Eigenschaft des Endprodukts und mit dem Korrekturmodell korrigierte Zielwerte der Kompensationselemente berechnet werden, um die zumindest eine werkstoffkundliche Eigenschaft in einen Zielbereich zu bringen.

2. Verfahren nach Anspruch 1, **dadurch gekennzeichnet, dass** die werkstoffkundlichen Eigenschaften Hollomon-Jaffe-Parameter, Härtbarkeitsindizes, ein oder mehrere Kohlenstoffäquivalente, Dehngrenze YS und Zugfestigkeit TS umfassen.

3. Verfahren nach Anspruch 1 oder 2, **dadurch gekennzeichnet, dass** die Analyse mittels Probenentnahme nach EN ISO 14284 durchgeführt wird.

4. Verfahren nach einem der vorhergehenden Ansprüche, **dadurch gekennzeichnet, dass** auf die Stahlherstellung folgende Nachbehandlungsschritte bis zum Erhalt des Endprodukts unverändert durchgeführt werden.

5. Verfahren nach einem der vorhergehenden Ansprüche, **dadurch gekennzeichnet, dass** das Prognosemodell und/oder Korrekturmodell unter Verwendung eines, mehrerer oder aller nachfolgender Modelle erzeugt wird: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

6. Verfahren nach einem der vorhergehenden Ansprüche, **dadurch gekennzeichnet, dass**, wenn eine werkstoffkundliche Eigenschaft betrachtet wird, der zumindest eine erfasste Ist-Wert der Analyse unter Heranziehung der Quadratwurzel der mittleren quadratischen Abweichung der werkstoffkundlichen Eigenschaft dem zumindest einen Prognosemodell und/oder Korrekturmodell zugeordnet wird, wobei für den zumindest einen Ist-Wert die Quadratwurzel der mittleren quadratischen Abweichung der werkstoffkundlichen Eigenschaft für alle Prognosemodelle und/oder Korrekturmodelle ermittelt wird und basierend auf den Ergebnissen jenes Prognosemodell und/oder Korrekturmodell mit der höchsten Genauigkeit gewählt wird.

7. Verfahren nach einem der vorhergehenden Ansprüche, **dadurch gekennzeichnet, dass**, wenn mehrere werkstoffkundliche Eigenschaften betrachtet werden, der zumindest eine erfasste Ist-Wert der Analyse unter Heranziehung des Mittelwerts und/oder der Summe der Quadratwurzeln der mittleren quadratischen Abweichungen der werkstoffkundlichen Eigenschaften dem zumindest einen Prognosemodell und/oder Korrekturmodell zugeordnet wird, wobei für den zumindest einen Ist-Wert der Mittelwert und/oder die Summe der Quadratwurzeln der mittleren quadratischen Abweichungen der werkstoffkundlichen Eigenschaften für alle Prognosemodelle und/oder Korrekturmodelle ermittelt wird und basierend auf den Ergebnissen jenes Prognosemodell und/oder Korrekturmodell mit der höchsten Genauigkeit gewählt wird.

8. Verfahren nach einem der vorhergehenden Ansprüche, **dadurch gekennzeichnet, dass** das Prognosemodell und/oder Korrekturmodell unter Verwendung einer multiplen linearen Regressionsanalyse erzeugt wird.

9. Verfahren nach einem der vorhergehenden Ansprüche, **dadurch gekennzeichnet, dass** die Zugfestigkeit TS und die Dehngrenze YS in einem auf multipler linearer Regressionsanalyse basierenden Prognose- und/oder Korrekturmodell gemäß den folgenden Formeln berechnet werden: $TS = {α}_{0} + {α}_{1} \times {X}_{1} + α \times {X}_{2} + ⋯ + {α}_{n} \times {X}_{n} ;$ $YS = {β}_{0} + {β}_{1} \times {X}_{1} + {β}_{2} \times {X}_{2} + ⋯ + {β}_{n} \times {X}_{n} ,$ wobei
α₀ und β₀ Basisfaktoren der Zugfestigkeit TS und Dehngrenze YS,
α₁- αₙ und β₁- βₙ Regressionskoeffizienten und
X₁-Xₙ Elementgehalte in Gew.-% sind.

10. Verfahren nach einem der vorhergehenden Ansprüche, **dadurch gekennzeichnet, dass** die Einflusselemente, deren Einfluss auf werkstoffkundliche Eigenschaften des Endprodukts kompensiert werden soll, eines, mehrere oder alle aus der Gruppe von Ni, Mo, Cu umfassen.

11. Verfahren nach Anspruch 10, **dadurch gekennzeichnet, dass** bei Ni-legierten Vergütungsstählen Mo und Cu, bei Mo-legierten Vergütungsstählen Ni und Cu und bei Ni-Mo-legierten Vergütungsstählen Cu Einflusselemente sind, wobei in diesen Fällen entsprechend die zuvor genannten Legierungselemente auch als Kompensationselemente geeignet sind.

12. Verfahren nach einem der vorhergehenden Ansprüche, **dadurch gekennzeichnet, dass** als Kompensationselemente eines, mehrere oder alle aus der Gruppe von C, Si, Mn, Cr ausgewählt werden.

13. Verfahren nach einem der vorhergehenden Ansprüche, **dadurch gekennzeichnet, dass** zur Berechnung des Kohlenstoffäquivalents in Abhängigkeit von Normen und/oder Spezifikationen einer bestimmten Vergütungsstahlgüte zumindest eine der folgenden Formeln verwendet wird: $\begin{matrix}CEV \left(Carbon Equivalent Value\right) = Gew . - % C + \frac{Gew . - % Mn}{6} + \\ \frac{Gew . - % Cr + Gew . - % Mo + Gew . - % V}{5} + \frac{Gew . - % Ni + Gew . - % Cu}{15} ;\end{matrix}$ $\begin{matrix}CET \left(Carbon Equivalent Thyssen\right) = Gew . - % C + \frac{Gew . - % Mn + Gew . - % Mo}{10} + \\ \frac{Gew . - % Cr + Gew . - % Cu}{20} + \frac{Gew . - % Ni}{40} ;\end{matrix}$ $\begin{matrix}Pcm \left(Critical Metal Parameter\right) = Gew . - % C + \frac{Gew . - % Si}{30} + \\ \frac{Gew . - % Mn + Gew . - % Cu + Gew . - % Cr}{20} + \frac{Gew . - % Mo}{15} + \frac{Gew . - % Ni}{60} + \frac{Gew . - % V}{10} + 5 \times Gew . - %B ,\end{matrix}$ wobei Gew.-%i der Gehalt des jeweiligen Elements i in Gew.-% ist.

14. Verfahren nach einem der vorhergehenden Ansprüche, **dadurch gekennzeichnet, dass** die Analyse zumindest in der ersten Schmelze des letzten primärmetallurgischen Aggregates einer Erzeugungsroute, insbesondere ab einem Abstich von Rohstahl, vor oder nach einer ersten Zugabe von Legierungselementen und zumindest nach einer zweiten Zugabe von Legierungselementen durchgeführt wird.

15. Verfahren nach einem der vorhergehenden Ansprüche, **dadurch gekennzeichnet, dass**, wenn ein erstes Element durch einen Einsatzstoff in einem Anteil eingebracht wird, der unterhalb eines gewünschten, durch die Ziellegierung definierten Zielbereichs liegt, das erste Element, sofern es ein gewünschtes Legierungselement ist, in dem Umfang zulegiert wird, der zum Erreichen des Zielbereichs notwendig ist, oder, wenn das erste Element durch einen Einsatzstoff in einem Anteil eingebracht wird, der bereits im Zielbereich entspricht, das erste Element nicht mehr zulegiert wird oder, wenn das erste Element durch einen Einsatzstoff in einem Anteil eingebracht wird, der über dem Zielbereich liegt, das erste Element durch einen verringerten Legierungsanteil eines zweiten ähnlich wirkenden Elements oder einen erhöhten Legierungsanteil eines gegenläufig wirkenden dritten Elements kompensiert wird.

16. Verfahren nach einem der vorhergehenden Ansprüche, **dadurch gekennzeichnet, dass** das verwendete Korrekturmodell eine Reihung der unterschiedlichen Kompensationselemente nach auflösungs- und ausbringungsspezifischen sowie ökonomischen Gesichtspunkten festlegt.

17. Verfahren nach einem der vorhergehenden Ansprüche, **dadurch gekennzeichnet, dass** die Ziellegierung die folgende Ziellegierungszusammensetzung in Gew.-% umfasst:
| | |
|---|---|
| C | 0,05-1,2 |
| Si | 0,001-1,9 |
| Mn | 0,1-3,5 |
zudem Eisen und unvermeidbare Verunreinigungen,
wobei lediglich optional eines, mehrere oder alle der folgenden Elemente in Gew.-% enthalten sind:
| | |
|---|---|
| P | ≤ 0,1 |
| S | ≤0,35 |
| Cr | 0,001-15 |
| Cu | 0,001-0,5 |
| Mo | 0,001-1,5 |
| Ni | 0,001-4,0 |
| N | 0,0001-0,05 |
| Sn | 0,001-0,03 |
| V | 0,001-0,4 |
| Nb | 0,001-0,1 |
| Ti | 0,001-0,08 |
| Al | 0,0005-0,1 |
| B | 0,0005-0,01. |

18. Steuersystem zur Herstellung von Vergütungsstählen für Ölfeldanwendungen mit spezifizierten werkstoffkundlichen Eigenschaften, welche durch eine Ziellegierung sichergestellt werden, und zur Durchführung des Verfahrens nach einem der vorhergehenden Ansprüche, **dadurch gekennzeichnet, dass** das Steuersystem zwei Komponenten umfasst: ein Prognosemodell und ein Korrekturmodell, wobei das Prognosemodell dazu ausgelegt ist, eine Wirkung von Einflusselementen und Kompensationselementen auf zumindest eine werkstoffkundliche Eigenschaften des Vergütungsstahls zu berechnen, und das Korrekturmodell dazu ausgelegt ist, korrigierte Zielwerte von Kompensationselementen zu berechnen, um die zumindest eine werkstoffkundliche Eigenschaft in einen Zielbereich zu bringen.

19. Steuersystem nach Anspruch 18, **dadurch gekennzeichnet, dass** das Prognosemodell und/oder Korrekturmodell unter Verwendung eines, mehrerer oder aller nachfolgender Modelle erzeugbar ist: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

20. Steuersystem nach Anspruch 18 oder 19, **dadurch gekennzeichnet, dass** die werkstoffkundlichen Eigenschaften Zugfestigkeit TS und Dehngrenze YS in einem auf multipler linearer Regressionsanalyse basierenden Prognose- und/oder Korrekturmodell gemäß den folgenden Formeln berechenbar sind: $TS = {α}_{0} + {α}_{1} \times {X}_{1} + α \times {X}_{2} + ⋯ + {α}_{n} \times {X}_{n} ;$ $YS = {β}_{0} + {β}_{1} \times {X}_{1} + {β}_{2} \times {X}_{2} + ⋯ + {β}_{n} \times {X}_{n} ,$ wobei
α₀ und β₀ Basisfaktoren der Zugfestigkeit TS und Dehngrenze YS,
α₁- αₙ und β₁- βₙ Regressionskoeffizienten und
X₁-Xₙ Elementgehalte in Gew.-% sind.

21. Steuersystem nach einem der Ansprüche 18 bis 20, **dadurch gekennzeichnet, dass** zur Berechnung des Kohlenstoffäquivalents in Abhängigkeit von Normen und/oder Spezifikationen einer bestimmten Vergütungsstahlgüte zumindest eine der folgenden Formeln verwendbar ist: $\begin{matrix}CEV \left(Carbon Equivalent Value\right) = Gew . - % C + \frac{Gew . - % Mn}{6} + \\ \frac{Gew . - % Cr + Gew . - % Mo + Gew . - % V}{5} + \frac{Gew . - % Ni + Gew . - % Cu}{15} ;\end{matrix}$ $\begin{matrix}CET \left(Carbon Equivalent Thyssen\right) = Gew . - % C + \frac{Gew . - % Mn + Gew . - % Mo}{10} + \\ \frac{Gew . - % Cr + Gew . - % Cu}{20} + \frac{Gew . - % Ni}{40} ;\end{matrix}$ $\begin{matrix}Pcm \left(Critical Metal Parameter\right) = Gew . - % C + \frac{Gew . - % Si}{30} + \\ \frac{Gew . - % Mn + Gew . - % Cu + Gew . - % Cr}{20} + \frac{Gew . - % Mo}{15} + \frac{Gew . - % Ni}{60} + \frac{+ Gew . - % V}{10} + 5 \times Gew . - %B ,\end{matrix}$ wobei Gew.-%i der Gehalt des jeweiligen Elements i in Gew.-% ist.

22. Steuersystem nach einem der Ansprüche 18 bis 21, **dadurch gekennzeichnet, dass** die Ziellegierung die folgende Ziellegierungszusammensetzung in Gew.-% umfasst:
| | |
|---|---|
| C | 0,05-1,2 |
| Si | 0,001-1,9 |
| Mn | 0,1-3,5 |
zudem Eisen und unvermeidbare Verunreinigungen,
wobei lediglich optional eines, mehrere oder alle der folgenden Elemente in Gew.-% enthalten sind:
| | |
|---|---|
| P | ≤ 0,1 |
| S | ≤0,35 |
| Cr | 0,001-15 |
| Cu | 0,001-0,5 |
| Mo | 0,001-1,5 |
| Ni | 0,001-4,0 |
| N | 0,0001-0,05 |
| Sn | 0,001-0,03 |
| V | 0,001-0,4 |
| Nb | 0,001-0,1 |
| Ti | 0,001-0,08 |
| Al | 0,0005-0,1 |
| B | 0,0005-0,01. |

23. Metallischer Hohlkörper, insbesondere nahtloses Stahlrohr für Ölfeldanwendungen aus einem Vergütungsstahl, hergestellt nach dem Verfahren nach einem der Ansprüche 1 bis 17.

## Geänderte Patentansprüche

### Geänderte Patentansprüche gemäss Regel 137(2) EPÜ.

1. Verfahren zur Herstellung von Vergütungsstählen für Ölfeldanwendungen mit spezifizierten werkstoffkundlichen Eigenschaften, welche durch eine Ziellegierung sichergestellt werden, wobei zumindest ein primärmetallurgisches Aggregat zu einer ersten Schmelze führt, wobei aus der ersten Schmelze zumindest eine Analyse von Gehalten der Elemente durchgeführt wird, wobei zumindest ein Ist-Wert der Schmelzanalyse an ein Steuersystem geleitet wird,
**dadurch gekennzeichnet, dass**
zwischen Einfluss- und Kompensationselementen unterschieden wird, wobei
Einflusselemente Elemente sind, welche einen Einfluss auf zumindest eine werkstoffkundliche Eigenschaft eines Endprodukts haben, und Kompensationselemente Elemente sind, die einen Einfluss auf die zumindest eine werkstoffkundliche Eigenschaft haben und den Einfluss der Einflusselemente kompensieren, wobei
die Einflusselemente, deren Einfluss auf werkstoffkundliche Eigenschaften des Endprodukts kompensiert werden soll, eines, mehrere oder alle aus der Gruppe von Ni, Mo, Cu umfassen, wobei
als Kompensationselemente eines, mehrere oder alle aus der Gruppe von C, Si, Mn, Cr ausgewählt werden, wobei
die werkstoffkundlichen Eigenschaften Hollomon-Jaffe-Parameter, Härtbarkeitsindizes, ein oder mehrere Kohlenstoffäquivalente, Dehngrenze YS und Zugfestigkeit TS umfassen, wobei
die Größe des Einflusses des zumindest einen Ist-Werts der jeweiligen Einfluss- und Kompensationselemente auf die zumindest eine werkstoffkundliche Eigenschaft des Endprodukts bekannt ist, wobei
der zumindest eine Ist-Wert bezüglich derjenigen Einfluss- und Kompensationselemente erfasst wird, welche einen Einfluss auf die zumindest eine werkstoffkundliche Eigenschaft des Endprodukts haben, wobei zur zumindest einen werkstoffkundlichen Eigenschaft des Endprodukts zumindest ein Kompensationselement ermittelt wird, welches die zumindest eine werkstoffkundliche Eigenschaft einstellt, wobei
eines oder mehrere Kompensationselemente in die Schmelze in einer Menge zulegiert werden, welche die zumindest eine werkstoffkundliche Eigenschaft in einen Zielbereich bringt und den Einfluss eines oder mehrerer Einflusselemente auf die zumindest eine werkstoffkundliche Eigenschaft kompensiert, und wobei
hierfür im Steuersystem zwei Komponenten verwendet werden: ein Prognosemodell und ein Korrekturmodell, wobei mit dem Prognosemodell der Einfluss der Einfluss- und Kompensationselemente auf die zumindest eine werkstoffkundliche Eigenschaft des Endprodukts und mit dem Korrekturmodell korrigierte Zielwerte der Kompensationselemente berechnet werden, um die zumindest eine werkstoffkundliche Eigenschaft in einen Zielbereich zu bringen.

2. Verfahren nach Anspruch 1, **dadurch gekennzeichnet, dass** die Analyse mittels Probenentnahme nach EN ISO 14284 durchgeführt wird.

3. Verfahren nach Anspruch 1 oder 2, **dadurch gekennzeichnet, dass** auf die Stahlherstellung folgende Nachbehandlungsschritte bis zum Erhalt des Endprodukts unverändert durchgeführt werden.

4. Verfahren nach einem der vorhergehenden Ansprüche, **dadurch gekennzeichnet, dass** das Prognosemodell und/oder Korrekturmodell unter Verwendung eines, mehrerer oder aller nachfolgender Modelle erzeugt wird: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

5. Verfahren nach einem der vorhergehenden Ansprüche, **dadurch gekennzeichnet, dass**, wenn eine werkstoffkundliche Eigenschaft betrachtet wird, der zumindest eine erfasste Ist-Wert der Analyse unter Heranziehung der Quadratwurzel der mittleren quadratischen Abweichung der werkstoffkundlichen Eigenschaft dem zumindest einen Prognosemodell und/oder Korrekturmodell zugeordnet wird, wobei für den zumindest einen Ist-Wert die Quadratwurzel der mittleren quadratischen Abweichung der werkstoffkundlichen Eigenschaft für alle Prognosemodelle und/oder Korrekturmodelle ermittelt wird und basierend auf den Ergebnissen jenes Prognosemodell und/oder Korrekturmodell mit der höchsten Genauigkeit gewählt wird.

6. Verfahren nach einem der vorhergehenden Ansprüche, **dadurch gekennzeichnet, dass**, wenn mehrere werkstoffkundliche Eigenschaften betrachtet werden, der zumindest eine erfasste Ist-Wert der Analyse unter Heranziehung des Mittelwerts und/oder der Summe der Quadratwurzeln der mittleren quadratischen Abweichungen der werkstoffkundlichen Eigenschaften dem zumindest einen Prognosemodell und/oder Korrekturmodell zugeordnet wird, wobei für den zumindest einen Ist-Wert der Mittelwert und/oder die Summe der Quadratwurzeln der mittleren quadratischen Abweichungen der werkstoffkundlichen Eigenschaften für alle Prognosemodelle und/oder Korrekturmodelle ermittelt wird und basierend auf den Ergebnissen jenes Prognosemodell und/oder Korrekturmodell mit der höchsten Genauigkeit gewählt wird.

7. Verfahren nach einem der vorhergehenden Ansprüche, **dadurch gekennzeichnet, dass** das Prognosemodell und/oder Korrekturmodell unter Verwendung einer multiplen linearen Regressionsanalyse erzeugt wird.

8. Verfahren nach einem der vorhergehenden Ansprüche, **dadurch gekennzeichnet, dass** die Zugfestigkeit TS und die Dehngrenze YS in einem auf multipler linearer Regressionsanalyse basierenden Prognose- und/oder Korrekturmodell gemäß den folgenden Formeln berechnet werden: $TS = {α}_{0} + {α}_{1} \times {X}_{1} + α \times {X}_{2} + ⋯ + {α}_{n} \times {X}_{n} ;$ $YS = {β}_{0} + {β}_{1} \times {X}_{1} + {β}_{2} \times {X}_{2} + ⋯ + {β}_{n} \times {X}_{n} ,$ wobei
α₀ und β₀ Basisfaktoren der Zugfestigkeit TS und Dehngrenze YS,
α₁- αₙ und β₁- βₙ Regressionskoeffizienten und
X₁-Xₙ Elementgehalte in Gew.-% sind.

9. Verfahren nach einem der vorhergehenden Ansprüche, **dadurch gekennzeichnet, dass** bei Ni-legierten Vergütungsstählen Mo und Cu, bei Mo-legierten Vergütungsstählen Ni und Cu und bei Ni-Mo-legierten Vergütungsstählen Cu Einflusselemente sind, wobei in diesen Fällen entsprechend die zuvor genannten Legierungselemente auch als Kompensationselemente geeignet sind.

10. Verfahren nach einem der vorhergehenden Ansprüche, **dadurch gekennzeichnet, dass** zur Berechnung des Kohlenstoffäquivalents in Abhängigkeit von Normen und/oder Spezifikationen einer bestimmten Vergütungsstahlgüte zumindest eine der folgenden Formeln verwendet wird: $\begin{matrix}CEV \left(Carbon Equivalent Value\right) = Gew . - % C + \frac{Gew . - % Mn}{6} + \\ \frac{Gew . - % Cr + Gew . - % Mo + Gew . - % V}{5} + \frac{Gew . - % Ni + Gew . - % Cu}{15} ; \\ CET \left(Carbon Equivalent Thyssen\right) = Gew . - % C + \frac{Gew . - % Mn + Gew . - % Mo}{10} + \\ \frac{Gew . - % Cr + Gew . - % Cu}{20} + \frac{Gew . - % Ni}{40} ;\end{matrix}$ $\begin{matrix}Pcm \left(Critical Metal Parameter\right) = Gew . - % C + \frac{Gew . - % Si}{30} + \\ \frac{Gew . - % Mn + Gew . - % Cu + Gew . - % Cr}{20} + \frac{Gew . - % Mo}{15} + \frac{Gew . - % Ni}{60} + \frac{Gew . - % V}{10} + 5 \times Gew . - %B ,\end{matrix}$ wobei Gew.-%i der Gehalt des jeweiligen Elements i in Gew.-% ist.

11. Verfahren nach einem der vorhergehenden Ansprüche, **dadurch gekennzeichnet, dass** die Analyse zumindest in der ersten Schmelze des letzten primärmetallurgischen Aggregates einer Erzeugungsroute, insbesondere ab einem Abstich von Rohstahl, vor oder nach einer ersten Zugabe von Legierungselementen und zumindest nach einer zweiten Zugabe von Legierungselementen durchgeführt wird.

12. Verfahren nach einem der vorhergehenden Ansprüche, **dadurch gekennzeichnet, dass**, wenn ein erstes Element durch einen Einsatzstoff in einem Anteil eingebracht wird, der unterhalb eines gewünschten, durch die Ziellegierung definierten Zielbereichs liegt, das erste Element, sofern es ein gewünschtes Legierungselement ist, in dem Umfang zulegiert wird, der zum Erreichen des Zielbereichs notwendig ist, oder, wenn das erste Element durch einen Einsatzstoff in einem Anteil eingebracht wird, der bereits im Zielbereich entspricht, das erste Element nicht mehr zulegiert wird oder, wenn das erste Element durch einen Einsatzstoff in einem Anteil eingebracht wird, der über dem Zielbereich liegt, das erste Element durch einen verringerten Legierungsanteil eines zweiten ähnlich wirkenden Elements oder einen erhöhten Legierungsanteil eines gegenläufig wirkenden dritten Elements kompensiert wird.

13. Verfahren nach einem der vorhergehenden Ansprüche, **dadurch gekennzeichnet, dass** das verwendete Korrekturmodell eine Reihung der unterschiedlichen Kompensationselemente nach auflösungs- und ausbringungsspezifischen sowie ökonomischen Gesichtspunkten festlegt.

14. Verfahren nach einem der vorhergehenden Ansprüche, **dadurch gekennzeichnet, dass** die Ziellegierung die folgende Ziellegierungszusammensetzung in Gew.-% umfasst:
| | |
|---|---|
| C | 0,05-1,2 |
| Si | 0,001-1,9 |
| Mn | 0,1-3,5 |
zudem Eisen und unvermeidbare Verunreinigungen,
wobei lediglich optional eines, mehrere oder alle der folgenden Elemente in Gew.-% enthalten sind:
| | |
|---|---|
| P | ≤ 0,1 |
| S | ≤0,35 |
| Cr | 0,001-15 |
| Cu | 0,001-0,5 |
| Mo | 0,001-1,5 |
| Ni | 0,001-4,0 |
| N | 0,0001-0,05 |
| Sn | 0,001-0,03 |
| V | 0,001-0,4 |
| Nb | 0,001-0,1 |
| Ti | 0,001-0,08 |
| Al | 0,0005-0,1 |
| B | 0,0005-0,01. |

15. Steuersystem zur Herstellung von Vergütungsstählen für Ölfeldanwendungen mit spezifizierten werkstoffkundlichen Eigenschaften, welche durch eine Ziellegierung sichergestellt werden, und zur Durchführung des Verfahrens nach einem der vorhergehenden Ansprüche, **dadurch gekennzeichnet, dass** das Steuersystem zwei Komponenten umfasst: ein Prognosemodell und ein Korrekturmodell, wobei das Prognosemodell dazu ausgelegt ist, eine Wirkung von Einflusselementen und Kompensationselementen auf zumindest eine werkstoffkundliche Eigenschaften des Vergütungsstahls zu berechnen, und das Korrekturmodell dazu ausgelegt ist, korrigierte Zielwerte von Kompensationselementen zu berechnen, um durch Zulegieren zumindest eines Kompensationselements die zumindest eine werkstoffkundliche Eigenschaft in einen Zielbereich zu bringen.

16. Steuersystem nach Anspruch 15, **dadurch gekennzeichnet, dass** das Prognosemodell und/oder Korrekturmodell unter Verwendung eines, mehrerer oder aller nachfolgender Modelle erzeugbar ist: lineare Regressionsmodelle, polynomiale Regressionsmodelle, Decision-Tree-Based Regressionsmodelle, Random-Forest-Regressionsmodelle, Nearest-Neighbour-Modelle, Neuronal-Network-Modelle, Support-Vector-Machine-Modelle, ADA-Boost-Modelle, Regression-Boost-Modelle, HIST-Boost-Modelle, XGBOOST-Modelle, Generalized-Additive-Modelle, Symbolic-Regression-Modelle.

17. Steuersystem nach Anspruch 15 oder 16, **dadurch gekennzeichnet, dass** die werkstoffkundlichen Eigenschaften Zugfestigkeit TS und Dehngrenze YS in einem auf multipler linearer Regressionsanalyse basierenden Prognose- und/oder Korrekturmodell gemäß den folgenden Formeln berechenbar sind: $TS = {α}_{0} + {α}_{1} \times {X}_{1} + α \times {X}_{2} + ⋯ + {α}_{n} \times {X}_{n} ;$ $YS = {β}_{0} + {β}_{1} \times {X}_{1} + {β}_{2} \times {X}_{2} + ⋯ + {β}_{n} \times {X}_{n} ,$ wobei
α₀ und β₀ Basisfaktoren der Zugfestigkeit TS und Dehngrenze YS,
α₁- αₙ und β₁- βₙ Regressionskoeffizienten und
X₁-Xₙ Elementgehalte in Gew.-% sind.

18. Steuersystem nach einem der Ansprüche 15 bis 17, **dadurch gekennzeichnet, dass** zur Berechnung des Kohlenstoffäquivalents in Abhängigkeit von Normen und/oder Spezifikationen einer bestimmten Vergütungsstahlgüte zumindest eine der folgenden Formeln verwendbar ist: $\begin{matrix}CEV \left(Carbon Equivalent Value\right) = Gew . - % C + \frac{Gew . - % Mn}{6} + \\ \frac{Gew . - % Cr + Gew . - % Mo + Gew . - % V}{5} + \frac{Gew . - % Ni + Gew . - % Cu}{15} ;\end{matrix}$ $\begin{matrix}CET \left(Carbon Equivalent Thyssen\right) = Gew . - % C + \frac{Gew . - % Mn + Gew . - % Mo}{10} + \\ \frac{Gew . - % Cr + Gew . - % Cu}{20} + \frac{Gew . - % Ni}{40} ;\end{matrix}$ $\begin{matrix}Pcm \left(Critical Metal Parameter\right) = Gew . - % C + \frac{Gew . - % Si}{30} + \\ \frac{Gew . - % Mn + Gew . - % Cu + Gew . - % Cr}{20} + \frac{Gew . - % Mo}{15} + \frac{Gew . - % Ni}{60} + \frac{Gew . - % V}{10} + 5 \times Gew . - %B ,\end{matrix}$ wobei Gew.-%i der Gehalt des jeweiligen Elements i in Gew.-% ist.

19. Steuersystem nach einem der Ansprüche 15 bis 18, **dadurch gekennzeichnet, dass** die Ziellegierung die folgende Ziellegierungszusammensetzung in Gew.-% umfasst:
| | |
|---|---|
| C | 0,05-1,2 |
| Si | 0,001-1,9 |
| Mn | 0,1-3,5 |
zudem Eisen und unvermeidbare Verunreinigungen,
wobei lediglich optional eines, mehrere oder alle der folgenden Elemente in Gew.-% enthalten sind:
| | |
|---|---|
| P | ≤ 0,1 |
| S | ≤0,35 |
| Cr | 0,001-15 |
| Cu | 0,001-0,5 |
| Mo | 0,001-1,5 |
| Ni | 0,001-4,0 |
| N | 0,0001-0,05 |
| Sn | 0,001-0,03 |
| V | 0,001-0,4 |
| Nb | 0,001-0,1 |
| Ti | 0,001-0,08 |
| Al | 0,0005-0,1 |
| B | 0,0005-0,01. |

20. Metallischer Hohlkörper, insbesondere nahtloses Stahlrohr für Ölfeldanwendungen aus einem Vergütungsstahl, hergestellt nach dem Verfahren nach einem der Ansprüche 1 bis 14.
